# EUROPEAN PATENT SPECIFICATION

(11) **EP 4 255 544 B1**
(45) Date of publication and mention of the grant of the patent: **27.05.2026**
(21) Application number: 21901296.0
(22) Date of filing: 30.11.2021
(51) Int. Cl.: A61M 16/10, A61M 16/12, A61M 16/20

(54) **THERMOGENIC AIRWAY MANAGEMENT DEVICE**
THERMOGENE ATEMWEGSVERWALTUNGSVORRICHTUNG
DISPOSITIF DE GESTION DE VOIE RESPIRATOIRE THERMOGÉNIQUE

(30) Priority: 01.12.2020 US 202063120119 P
(43) Date of publication of application: 11.10.2023
(73) Proprietor: The Government of The United States, as represented by The Secretary of The Army, Fort Detrick, MD 21702 (US)
(72) Inventor: PIERCE, Bradley T., Bethesda, Maryland 20814 (US); STEVENS, Ryan A., Bethesda, Maryland 20814 (US); TILLEY, Laura C., Bethesda, Maryland 20814 (US)
(74) Representative: Seyer & Nobbe Patentanwälte PartmbB
(86) International application number: PCT/US2021/061076
(87) International publication number: WO 2022/119790

(56) References cited:
- WO-A1-2009/118718
- WO-A1-2012/080923
- WO-A1-2019/112447
- WO-A1-2020/171720
- US-A- 4 588 425
- US-A1- 2011 253 136
- US-A1- 2011 253 136
- US-A1- 2015 151 073
- US-A1- 2016 374 592
- US-A1- 2017 216 552
- US-A1- 2019 344 038
- US-A1- 2020 352 474
- US-B2- 7 617 824
- US-B2- 8 905 019

## Description

This PCT international application claims priority to U.S. Serial No. 63/120,119, filed in the U.S. Patent and Trademark Office on 1 December 2020.

### TECHNICAL FIELD

The present invention is directed to a device for preventing and/or treating hypothermia in a patient.

### BACKGROUND

Hypothermia is a dangerous condition for the human body that can sometimes lead to death. Hypothermia may be caused by prolonged exposure to cold temperatures. Additionally, hypothermia may be caused by trauma, such as due to a body's reaction to internal bleeding. Traditional treatment of hypothermia in a pre-hospital setting, such as on a battlefield or in the mountains, includes wrapping the victim in blankets, body to body heat transfer, and the like. In addition, transporting a patient from point of injury to a hospital may be a significant contributor to heat loss due to further exposure. These traditional techniques are slow and oftentimes ineffectual for certain victims, such as those suffering from trauma.

US2011253136A1 discloses an apparatus for treatment of respiratory conditions such as the conditions related to sleep disordered breathing (SDB), allergy induced upper airway obstruction or early viral infection of the upper airway.

### SUMMARY OF INVENTION

The invention is defined in the appended independent claim 1. Preferred embodiments are matter of the dependent claims.

In an embodiment, the invention provides a device for treating and/or preventing hypothermia in a patient comprising:
an opening for ambient air;
a temperature sensor configured to measure a temperature of the ambient air;
at least one battery;
a furnace section comprising a furnace, a heating element configured to heat air in the furnace section, a temperature sensor to determine a temperature of air in the furnace section, and an insulator in which the furnace is housed;
a valve configured to mix ambient air with pre-heated furnace air to form output air at an output air temperature based at least in part on data from the temperature sensor; and
an output for providing the output air to a patient.

In an embodiment, the heating element is characterized by heating wires, heating coils, or wire mesh.

In an embodiment further to any of the previous embodiments, the device is further characterized by a humidity sensor configured to measure a humidity of the ambient air.

In an embodiment further to any of the previous embodiments, the device is further characterized by a pitot system comprising a static pressure sensor and a dynamic pressure sensor configured to measure the static and dynamic pressures of the ambient air.

In an embodiment further to any of the previous embodiments, the device is further characterized by a valve or sliding plunger configured to seal an exhalation port during inhalation of the patient.

In an embodiment further to any of the previous embodiments, the device is further characterized by a valve or sliding plunger is configured to seal the device during exhalation of the patient and open an exhalation port.

In an embodiment further to any of the previous embodiments, the device is further characterized by an exhalation port having at least one switch or flap that is openable and closeable based at least in part on data from a dynamic pressure sensor.

In an embodiment further to any of the previous embodiments, the device is further characterized by a removably attachable humidifier cartridge configured to provide a liquid to the furnace section.

In an embodiment further to any of the previous embodiments, the device is further characterized by a humidifier cartridge comprising an ultrasonic transducer configured to spray mist into the furnace section.

In an embodiment further to any of the previous embodiments, the device is further characterized by a humidifier cartridge comprising at least one channel for the liquid and a spring-loaded plunger configured to provide a constant pressure to the liquid.

In an embodiment further to any of the previous embodiments, the device is further characterized by a humidifier cartridge comprising a heating element or wire configured to prevent the liquid from freezing.

In an embodiment further to any of the previous embodiments, the device is further characterized by a carbon dioxide sensor in an output section.

In an embodiment further to any of the previous embodiments, the device is further characterized by an output section having an output valve and/or output spring configured to maintain a positive end-expiatory pressure in lungs of the patient.

In an embodiment further to any of the previous embodiments, the device is further characterized by one or more output air sensors configured to measure a temperature and/or a humidity of the output air.

In an embodiment further to any of the previous embodiments, the device is further characterized by another temperature sensor that is configured to measure a temperature of the air in the furnace section.

In an embodiment further to any of the previous embodiments, the device is further characterized by a first battery configured to provide power to one or more components of the device and a second battery configured to charge the first battery.

In an embodiment further to any of the previous embodiments, the device is further characterized by one or more processors; and one or more computer-readable media storing instructions executable by the one or more processors, wherein the instructions program the one or more processors to receive sensor data associated with one or more sensors; and determine an amount to open the valve to mix furnace air with ambient air based at least in part on sensor data, wherein the furnace air heats the ambient air to an output air temperature associated with the output air.

In an embodiment further to any of the previous embodiments, the device is further characterized by a display, wherein the instructions further program the one or more processors to cause a user interface to be presented via the display of the device, the user interface enabling an operator of the device to input a property of the air; and receive, via the user interface, the output air temperature associated with the output air.

In an embodiment further to any of the previous embodiments, the device is further characterized by instructions to program the one or more processors to determine a cycle associated with providing the output air to the patient, wherein the cycle comprises an inhalation of the air and an exhalation of at least a portion of the air from the patient; determine a first period of time associated with the inhalation; and cause an indication of the first period of time to be presented to an operator via a user interface of the device.

Further, there is disclosed, but not separately claimed, a method for treating and/or preventing hypothermia in a patient with a device comprising an opening for ambient air; a temperature sensor configured to measure a temperature of the ambient air; at least one battery; a furnace section comprising a heating element configured to heat air in the furnace section; a valve configured to mix ambient air with heated furnace air to form output air at an output air temperature based at least in part on data from the temperature sensor; and an output for providing the output air to a patient, the method characterized by receiving ambient air via the opening; receiving temperature data from the temperature sensor; heating air in the furnace section by providing power from the at least one battery to the heating element; opening the valve to mix heated air in a furnace section with ambient air based at least in part on the ambient air temperature data to form output air at an output air temperature; and directing the output air to a patient.

In an embodiment, the method is further characterized by receiving pressure data from a pitot system and humidity data from a humidity sensor; and adjusting the output air based at least in part on the pressure data and humidity data.

In an embodiment further to any of the previous method embodiments, the method is further characterized by receiving temperature data for the heated air in the furnace section from a second temperature sensor; and opening the valve to mix heated air in a furnace section with ambient air based at least in part on the ambient air temperature data and furnace air temperature data to form the output air.

In an embodiment further to any of the previous method embodiments, the method is further characterized by causing a user interface to be presented via a display of the device, the user interface enabling an operator of the device to input a property of the air; and receiving, via the user interface, an output air temperature associated with the output air.

In an embodiment further to any of the previous method embodiments, the method is further characterized by ejecting an amount of liquid from a humidifier cartridge into the furnace section based on sensor data from at least one of a temperature sensor or humidity sensor for the ambient air.

In an embodiment further to any of the previous method embodiments, the method is further characterized by determining a first humidity associated with the output air to be provided to the patient; determining a second humidity associated with the ambient air; determining an amount of liquid to eject into the furnace section based at least in part on the first humidity and the second humidity; and causing an ultrasonic transducer of a humidifier cartridge to eject the amount of water into the furnace section.

In an embodiment further to any of the previous method embodiments, the method is further characterized by receiving, via a user interface of the device, an input comprising patient data; determining a volume of the air to provide to the patient in a cycle of the output air based at least in part on the patient data; and determining an amount and/or rate at which to open the valve based at least in part on the volume of the air.

In an embodiment further to any of the previous method embodiments, the method is further characterized by receiving an input corresponding to a positive end-expiratory pressure for the patient; and causing an output valve and/or an output spring to maintain the positive end-expiratory pressure during an exhalation of at least a portion of the air from the patient.

In an embodiment further to any of the previous method embodiments, the method is further characterized by heating air in the furnace section by heating ambient air routed to the furnace section during a discharge cycle of a first battery.

In an embodiment further to any of the previous method embodiments, the method is further characterized by sealing an exhalation port during inhalation of the patient via a valve or sliding plunger.

In an embodiment further to any of the previous method embodiments, the method is further characterized by sealing the device during exhalation of the patient and opening an exhalation port via a valve or sliding plunger.

In an embodiment further to any of the previous method embodiments, the method is further characterized by determining that a first battery of the device is configured to provide power to one or more components of the device via a first circuit at a first time; causing the first circuit to be closed at the first time, wherein the first circuit electrically couples the first battery to the one or more components of the device; determining a second time associated with charging the first battery via a second circuit; and causing the first circuit to be open and the second circuit to be closed based at least in part on a second time, wherein the second circuit electrically couples the first battery to a second battery, the second battery being configured to charge the first battery via the second circuit.

In an embodiment further to any of the previous method embodiments, the method is further characterized in that the first circuit electrically couples one or more cells of the first battery to the one or more components of the device in series, and the second circuit electrically couples the one or more cells of the first battery to the second battery in parallel.

### BRIEF DESCRIPTION OF THE DRAWINGS

The detailed description is described with reference to the accompanying figures, which are presented for a better understanding of the aspects and embodiments of the present application. In the figures, the left-most digit(s) of a reference number usually identifies the figure in which the reference number first appears. The same reference numbers in different figures indicate similar or identical items. The figures are merely exemplary to illustrate certain features that may be used singularly or in combination with other features and the present application should not be limited to the embodiments shown.
FIG. 1 illustrates a front perspective view of an example thermogenic airway management device configured to modify at least one property associated with ambient air to prevent or treat hypothermia.
FIG. 2 illustrates a rear perspective view of the example thermogenic airway management device described with respect to FIG. 1.
FIG. 3A illustrates an exploded view of example components of an example thermogenic airway management device.
FIG. 3B illustrates an exploded view of example components of another embodiment of the thermogenic airway management device.
FIG. 4A illustrates a cross-sectional view of an example thermogenic airway management device.
FIG. 4B illustrates a cross-sectional view of an example of another embodiment of the thermogenic airway management device.
FIG. 5 illustrates an example user interface associated with a thermogenic airway management device configured for receiving one or more inputs corresponding to one or more properties of air to be output via the thermogenic airway management device.
FIG. 6A illustrates an example chart of power cycles associated with a first battery providing power to a device based on the power cycles.
FIG. 6B illustrates an example chart of charging cycles associated with a second battery charging the first battery based on the power cycles.
FIG. 7 is a flow diagram illustrating an example process for modifying a temperature of ambient air to output air from a device at a particular temperature based on an input via a user interface.
FIG. 8 is a flow diagram illustrating an example process for modifying a humidity of ambient air to output air from a device at a particular humidity based on an input via a user interface.
FIG. 9 is a flow diagram illustrating an example process for outputting a particular amount of air from a device based on an input via a user interface.
FIG. 10 is a flow diagram illustrating an example process for modifying a setting associated with an output valve of a device to maintain a positive end-expiratory pressure in a patient configured to receive air via the device.
FIG. 11 is a flow diagram illustrating an example process for modifying a circuitry of a battery from a first circuit associated with discharging the battery to power a device to a second circuit associated with charging the battery based on a power cycle of a device.
FIG. 12 depicts an example block diagram illustrating a system schematic for providing thermogenic emergency airway management, as discussed herein.

### DETAILED DESCRIPTION

The following detailed description is presented to enable any person skilled in the art to make and use the subject of the application. For purposes of explanation, specific nomenclature is set forth to provide a thorough understanding of the present disclosure. However, it will be apparent to one skilled in the art that these specific details are not required to practice the subject of the application. Descriptions of specific applications are provided only as representative examples. The present application is not intended to be limited to the embodiments shown but is to be accorded the widest possible scope consistent with the principles and features disclosed herein.

This disclosure is generally directed to a system for thermogenic airway emergency management (STEAM) configured to prevent and/or treat hypothermia in a patient. In an embodiment, a STEAM device may be configured to be used in a pre-hospital setting, such as on a battlefield, in the mountains, onboard a ship (e.g., for drowning victims, etc.), in an ambulance, and the like. The STEAM device may be configured to receive input ambient air and modify one or more properties of the ambient air to output air at a temperature and/or humidity associated with the prevention and/or treatment of hypothermia. For example, the STEAM device may receive cold, dry ambient air and may be configured to modify the temperature and humidity of the ambient air to output warm (e.g., 100° Fahrenheit (F), 39 °Celsius (C), etc.), humid air to a patient. The output temperature may be safely limited to a maximum temperature to avoid thermal injuries identified by medical literature.

Traditional techniques for treating patients for hypothermia and/or trauma in a pre-hospital setting may include wrapping a patient in blankets, body to body heat transfer, and the like. However, these traditional techniques are slow and oftentimes ineffectual for certain patients, such as those suffering from trauma or severe hypothermia. These patients, if unable to quickly get access to a hospital setting for advanced treatment, may suffer exacerbated injuries or death due to the delayed advanced treatment. The STEAM device described herein provides a means by which patients may receive advanced treatment for hypothermia in the pre-hospital setting. Accordingly, the STEAM device described herein may significantly decrease mortality rates in the pre-hospital setting, saving lives in a myriad of environments, such as austere environments, sub-zero degree climates, on the battlefield, in the mountains, and near water, to name just a few.

The apparatuses and techniques described herein may be implemented in a number of ways. Example implementations are provided below with reference to the following figures.

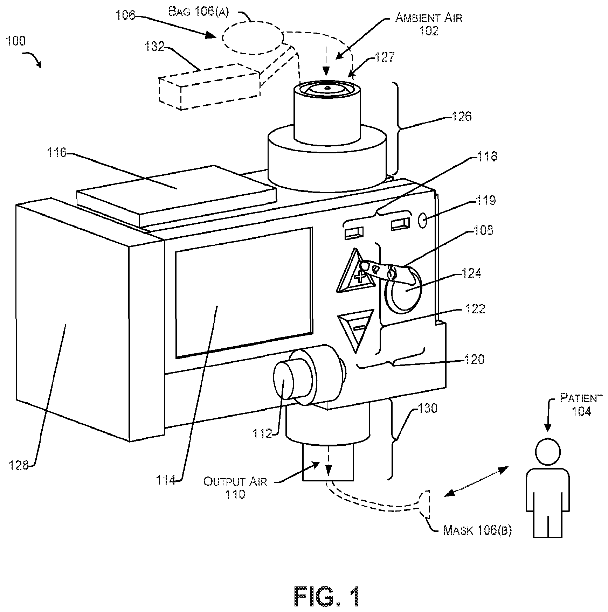

FIG. 1 is a front perspective view of a thermogenic airway emergency management device 100 (STEAM device 100) configured to modify at least one property associated with ambient air 102 to prevent or treat hypothermia in a patient 104. The patient 104 may be conscious or unconscious. For example, the patient may be intubated with an endotracheal tube placed. For another example, the patient 104 may be conscious and/or ambulatory.

In an embodiment, the STEAM device 100 may be coupled to the patient 104 via a ventilator mask. In such an embodiment, the STEAM device 100 may be configured to operate as a mechanical ventilator for the patient 104 in the pre-hospital setting. As illustrated in FIG. 1, the STEAM device 100 may be coupled to the patient 104 via a bag valve mask 106 (BVM 106). The BVM 106 may include at least two components, a bag 106(a) and a mask 106(b). In an embodiment, the mask 106(b) may represent the ventilator mask (e.g., include a mask that is compatible with a positive pressure air source). Further, an operator 108 (represented in FIG. 1 by a finger of a user) of the STEAM device 100 may manually assist the patient in breathing output air 110 from the STEAM device 100 based on a breathing cycle (e.g., one breath every five to six seconds, etc.).

The STEAM device 100 may include a power controller 112, enabling the operator 108 to turn the STEAM device 100 on and off. In an embodiment, the power controller 112 may include an on/off switch. As illustrated in FIG. 1, the power controller 112 includes a rotary knob. In an embodiment, the rotary knob of the power controller 112 may include a dimming function, enabling the operator 108 to modify a brightness of a front display 114 and/or a top display 116. For example, the operator 108 may dim the front display 114 and/or the top display 116 for enhanced viewing at night.

In an embodiment, the front display 114 and/or the top display 116 may provide a means by which the operator 108 may interact with the STEAM device 100, such as via a user interface. The front display 114 and/or the top display 116 may include a liquid crystal display, a plasma display, a light-emitting diode display, an OLED (organic light emitting diode) display, an electronic paper display, or any other suitable display technology. In an embodiment, the front display 114 and/or the top display 116 may have a touch sensor associated therewith to provide a touchscreen display configured to receive touch inputs for enabling interaction with a graphical user interface (referred to herein as a user interface) presented on the front display 114 and/or the top display 116.

As illustrated in FIG. 1, the STEAM device 100 includes the front display 114 and the top display 116. In an embodiment, the STEAM device 100 may include the front display 114 or the top display 116. In an embodiment not shown, the STEAM device 100 may not include the front display 114 or the top display 116. In an embodiment, in addition or alternative to the front display 114 or the top display 116, the STEAM device 100 may include one or more indicator lights 118. In an embodiment, the indicator lights 118 may provide an indication of when the STEAM device 100 is ready for operation. In at least one example, the indicator lights 118 may provide an indication of when the output air 110 is (or should be) provided to the patient 104. Additionally or alternatively, the STEAM device 100 may include a speaker 119 configured to output an aural signal indicating when the STEAM device 100 is ready for operation. For example, the aural signal may include a beep indicating when to squeeze the bag 106(a) of the BVM 106 and/or a voice signal saying "breathe," squeeze," or the like.

In an embodiment, the output air 110 may be provided to the patient automatically when the STEAM device 100 is operated in a ventilator mode (e.g., as a ventilator). Thus, the indicator lights 118 may indicate whether the output air 110 is being provided to the patient 104. For example, a first indicator light 118 may include a red light indicating that the output air 110 is not being provided to the patient 104 and a second indicator light 118 may include a green light indicating that the output air 110 is being provided to the patient 104.

In an embodiment, the STEAM device 100 may be operated additionally and/or alternatively to the ventilator mode, in a manual mode. In the manual mode, the operator 108 may utilize the BVM 106 to provide output air 110 to the patient 104. Further, the indicator lights 118 may provide an indication for the operator 108 to squeeze the bag 106(a) of the BVM 106 to provide the output air 110 to the patient 104. For example, a first indicator light 118 may include a red light indicating to not squeeze the bag 106(a) and a second indicator light 118 may include a green light indicating when to squeeze the bag 106(a). Though illustrated as having two indicator lights 118, this is not intended to be limiting and the STEAM device 100 may include a greater or lesser number of indicator lights 118. For example, the STEAM device 100 may include a single indicator light including a single-color bulb configured to provide an indication to the operator 108 of when to provide output air 110 to the patient 104. For another example, the STEAM device 100 may include a single indicator light 118 with different colored bulbs, such as red, yellow, and green bulbs, each color providing an indication to the operator 108 regarding a status of the STEAM device 100 and/or an indication of a breathing cycle, such as that the operator 108 should not provide output air 110, should prepare to provide output air 110, and should provide output air 110, respectively.

In an embodiment, the operator 108 may additionally or alternatively receive the indication of the status of the STEAM device 100 and/or an indication of the breathing cycle via one or more of the front display 114 or the top display 116. In at least one example, the top display 116 may be configured to display information regarding the status of the STEAM device 100, such as when the output air 110 is ready to be provided to the patient 104, and/or the indication of the breathing cycle, such as when the patient 104 is ready to receive the output air 110. For example, the top display 116 may be red when the patient 104 is not ready to receive output air 110, may turn yellow a period of time (e.g., one second, 1.5 seconds, etc.) before the patient is ready to receive the output air 110, such as to provide the operator 108 with a warning prior to operation, and may turn green when the patient 104 is ready to receive the output air 110. For another example, the top display 116 may provide a countdown timer, counting down to a time associated with providing the output air 110 to the patient.

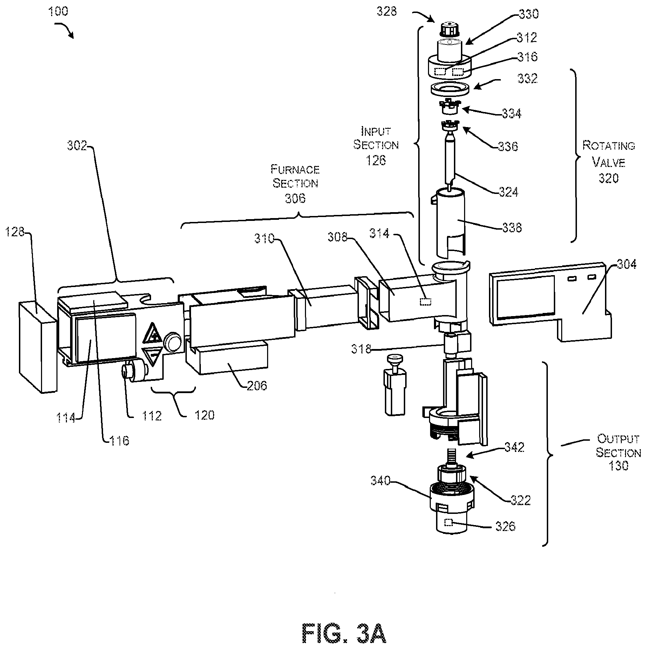

In an embodiment, the top display 116 and/or the front display 114 may provide an indication of a temperature and/or a humidity of the ambient air 102, air in the furnace (see FIGS. 3A-4B), and/or the output air 110. As will be discussed in greater detail below, a computing system of the STEAM device 100 may receive sensor data from one or more sensors of the STEAM device 100. A computing system of the STEAM device 100 may determine the temperature(s) and/or pressure(s) of the ambient air 102, the furnace air, and/or the output air 110 based on the sensor data and may cause the temperature(s) and/or pressure(s) to be presented on the front display 114 and/or the top display 116.

In an embodiment, the computing system may additionally be configured to determine a pressure of the ambient air 102, a density of the ambient air 102, a pressure altitude associated with the STEAM device 100 (e.g., pressure altitude associated with a current location of the STEAM device 100), and/or a density altitude associated with the STEAM device 100. In an embodiment, the computing system may cause one or more of the pressure of the ambient air 102, the density of the ambient air 102, the pressure altitude, and/or the density altitude to be presented via the top display 116 and/or the front display 114. Thus, the STEAM device 100 may be configured to operate as a stand-alone weather station.

In an embodiment, the top display 116 may be configured to provide one or more of an indication of the status of the STEAM device 100 and/or the indication of the breathing cycle, one or more parameters (e.g., temperature, pressure, humidity, etc.) of the ambient air, one or more parameters of the output air 110, and/or one or more altitudes associated with a location of the STEAM device 100, and the front display 114 may be configured to display one or more settings associated with the STEAM device 100. The setting(s) may include a desired output temperature, humidity, and/or volume associated with the output air 110, and/or a positive end-expiratory pressure associated with the STEAM device 100. In an embodiment, one or more of the settings may be pre-determined, such as that stored in a memory of the computing system. In such an embodiment, the pre-determined settings may be presented to the operator 108 via the front display 114 and/or the top display 116.

In an embodiment, one or more of the settings may be input by the operator 108, such as via a user interface. In such an embodiment, the computing system may receive an input via the user interface including one or more of the settings and may store the setting(s) in a memory. In an embodiment, the input may include a modification to one or more default settings associated with the STEAM device 100. Further, the one or more default settings may be stored in the memory of the STEAM device 100.

In an embodiment, the input may be received via a control panel 120 of the STEAM device 100. As illustrated in FIG. 1, the control panel 120 includes adjustment buttons 122 and/or a select button 124. In an embodiment, the adjustment buttons 122 may enable the operator 108 to scroll through the setting(s). In an embodiment, the operator 108 may select a setting for modification via the select button 124. In an embodiment, the operator 108 may increase or decrease a value associated with the selected setting utilizing the adjustment button 122. In an embodiment, the operator 108 may select a displayed value utilizing the select button 124.

In an embodiment, the input may be received via a touchscreen of the front display 114 and/or the top display 116. In such an embodiment, the touchscreen may enable the operator 108 to input one or more settings of the STEAM device 100. In at least one example, the input may include a desired temperature and humidity for the output air 110. Based at least in part on the input and/or one or more default settings, the STEAM device 100 may process ambient air 102 to output the output air 110 substantially at the desired temperature and humidity (e.g., within a threshold temperature (e.g., +/- 1°F, 0.5°C, etc.), within a threshold humidity (e.g., 0.5%, 1%, etc.)).

In an embodiment, the STEAM device 100 may be configured to output a particular volume of output air 110 (e.g., 500 milliliters (mL), 16 ounces, etc.). In an embodiment, the volume may be pre-determined, such as based on an average tidal volume associated with the breath. In an embodiment, the volume may be determined based on an input via the control panel and/or the touchscreen. In an embodiment, the input may include patient data. In such an embodiment, the computing system may be configured to determine the volume of output air 110 based on the patient data. The patient data may include at least one of a number of lungs of the patient, an age (e.g., adult, child, particular age, etc.), gender, height, weight, body temperature, or medical condition of the patient (e.g., hypothermia, trauma, etc.).

In an embodiment, the STEAM device 100 may be configured to maintain a positive end-expiratory pressure (PEEP) in the lungs (or lung) of the patient 104. In an embodiment, the STEAM device 100 may maintain a pre-determined positive end-expiratory pressure. In an embodiment, the positive end-expiratory pressure may be determined based on an input via the control panel 120 and/or the touchscreen. In such an embodiment, the computing system may adjust an output valve (e.g., positive end-expiratory pressure valve) to maintain the positive end-expiratory pressure in the lungs based on the input. Alternatively, or in addition, a positive end-expiratory pressure may be set by a mechanical input (e.g., dial, switch, button, knob) that is separate from input via the control panel 120 and/or a display touchscreen.

As discussed above, the STEAM device 100 may be configured to input ambient air, process the ambient air 102 to generate output air 110 at a determined temperature and/or humidity. In an embodiment, the temperature may be pre-defined, such as that stored in the memory of the STEAM device 100. In an embodiment, the temperature may be adjustable, such as by the operator 108 (for example, in a range of 98.6 °F to 104 °F, 37 °C to 40 °C). In an embodiment, the computing system of the STEAM device 100 may receive an input corresponding to a temperature for the output air 110. The computing system of the STEAM device 100 may determine an amount of pre-heated air to mix with ambient air 102 based on the input corresponding to the temperature.

In an embodiment, the computing system may determine the amount of pre-heated air to mix with the ambient air 102 to generate the output air 110 based on sensor data received from one or more sensors of the STEAM device 100. The sensor(s) may measure properties of air in the STEAM device 100 at various stages of processing (e.g., before heating (e.g., ambient), in a furnace, after heating, etc.). In an embodiment, STEAM device 100 may include sensor(s) in an input section 126 to measure at least one of temperature, humidity, pressure, or velocity associated with the ambient air 102 flowing into the STEAM device 100. In an embodiment, the ambient air 102 may flow into the STEAM device 100 based in part on a compression of the bag 106(a) (e.g., operator 108 squeezing the bag 106(a)). In such an embodiment, the ambient air 102 may be pushed into the input section. In an embodiment, the ambient air 102 may flow into the input section 126, such as via an opening 127, based on an input from a positive pressure air source 132 (e.g., forced air, compressed air, bag valve mask, etc.). In such an embodiment, the positive pressure air source 132 may be coupled to the STEAM device 100 and may cause the STEAM device 100 to operate as a mechanical ventilator.

In an embodiment, the sensor(s) in the input section 126 may include at least one of a temperature sensor, humidity sensor, a pitot system, or any combination thereof. In an embodiment, the temperature and the humidity of the ambient air 102 may be determined based on data from a single sensor or separate sensors. The pitot system may be configured to determine the static pressure of the ambient air 102 and the velocity of the ambient air 102 as it flows into the input section 126. In an embodiment, the computing system may utilize the temperature, humidity, pressure, and velocity of the ambient air 102 to determine an amount of pre-heated air to mix therewith to generate the output air 110 at the determined temperature.

In an embodiment, the STEAM device 100 may include a furnace section (FIGS. 3A-4B) including a heating element (e.g., heating wires, heating coils, resistance wires, wire mesh, etc.) configured to heat air in a furnace, for example, up to about 500 °F (260 °C). Any appropriate heating element may be used; however, the non-limiting embodiment of heating wires is discussed below. In at least one example, the heating element may include a Nichrome resistance wire mesh configured to transform electric potential energy into thermal energy to heat up the air as it passes over the wire mesh. In an embodiment, the computing system may determine an amount of energy to apply to the wires to heat air in the furnace based in part on the temperature of the ambient air 102. In an embodiment, the furnace may include a volume of, for example, about 10-40 milliliters. In at least one example, the furnace may hold about 10 milliliters of air. In at least one example, the furnace may hold about 15 milliliters of air. In at least one example, the furnace may hold about 20 milliliters of air. In at least one example, the furnace may hold about 25 milliliters of air. In at least one example, the furnace may hold about 30 milliliters of air. In an embodiment, the furnace section may include a temperature sensor to determine a temperature of the air in the furnace section. In such an embodiment, the computing system may utilize the temperature in the furnace section to determine the amount of furnace-heated air to mix with the ambient air 102 to generate the output air 110 at the determined temperature.

In an embodiment, the STEAM device 100 may include a humidifier cartridge 128 configured to provide mist (e.g., water) to the furnace section to increase the humidity of the air located therein. Any size humidifier cartridge may be used depending upon desired operational requirements. In an embodiment, the humidifier cartridge 128 may include a disposable cartridge including, for example, about 10-40 milliliters of sterilized water. In such an embodiment, the humidifier cartridge 128 may be detachably coupled to the STEAM device 100. In at least one example, the humidifier cartridge 128 may include a capacity of about 10 milliliters of water. In at least one example, the humidifier cartridge 128 may include a capacity of about 20 milliliters of water. In at least one example, the humidifier cartridge 128 may include a capacity of about 30 milliliters of water. In at least one example, the humidifier cartridge 128 may include a capacity of about 40 milliliters of water. In an embodiment, the humidifier cartridge 128 may include an ultrasonic humidifying element configured to spray a mist into the furnace section, to increase a humidity of the air. In at least one example, the humidifier cartridge 128 may include an ultrasonic piezoelectric transducer that is configured to vibrate at, for example, about 110 kilohertz-115 kilohertz to eject a fine mist into the furnace section.

In an embodiment, the computing system may determine an amount of mist to eject into the furnace section based on the humidity of the ambient air 102 measured in the input section 126 and a desired humidity of the output air 110. In an embodiment, the desired humidity of the output air 110 may be in a range from 0 to 100%. In an embodiment, the computing system may be programmed to determine an amount of mist to eject to increase humidity of the ambient air to about 10-100%, to generate the output air 110 to be provided to the patient 104. In such an embodiment, the input humidity may be about 60% humidity. In such an embodiment, the input humidity may be about 70% humidity. In such an embodiment, the input humidity may be about 80% humidity. In such an embodiment, the input humidity may be about 90% humidity. In such an embodiment, the input humidity may be up to about 100% humidity. In such an embodiment, the input humidity may be 100% humidity. In an embodiment, based on the input humidity and the humidity of the ambient air 102, the computing system may utilize pulse width modulation to provide energy to a transducer associated with the humidifier cartridge 128 to eject the amount of mist into the furnace section. It is important that moisture added to the air does not exceed the air's capacity to hold moisture, to prevent unevaporated water droplets from entering a patient's lungs, which may result in infections such as pneumonia.

In an embodiment, the STEAM device 100 may include one or more output air 110 sensors configured to measure the temperature and/or humidity of the output air 110. In an embodiment, the computing system may utilize the temperature and/or humidity of the output air 110 to determine one or more adjustments to the amount of furnace air to mix with ambient air 102 and/or the amount of mist to eject into the furnace section.

In an embodiment, the computing system may mix the furnace air with the ambient air 102 to generate the output air 110 at the determined temperature and/or humidity. In an embodiment, the computing system may control the mix via a valve, rotating valve, sliding valve, plunger, or any other appropriate apparatus that is openable and closeable to air flow. For simplicity, the non-limiting embodiment of a rotating valve is discussed below. The valve may prevent potentially flammable matter from the patient (e.g., sputum, blood, mucus) from entering the furnace section, where it might combust in an oxygen-rich environment. In an embodiment, the computing system may determine an amount to open the rotating valve based at least in part on a difference between the temperature of the ambient air 102 and the determined temperature of the output air 110. In an embodiment, the computing system may control a flow (rate) of furnace air from the furnace section, such as to prevent overheating the output air 110. Thus, the computing system may protect the patient from burns caused by overheated air. In an embodiment, the flow rate may be monitored by a sensor located in or proximate the output section 130. In an embodiment, the sensor may be located proximate the rotating valve.

As discussed above, the computing system may be configured to control a volume of output air 110 provided to the patient 104. In an embodiment, the volume of output air 110 may be pre-determined, such as based on a default setting. In an embodiment, the volume of output air 110 may be determined based on an input provided via a user interface. In an embodiment, the input may include patient data associated with the patient. The patient data may include at least one of a number of lungs, an age, gender, whether the patient 104 is a child or adult, a height, a weight, a body temperature, or a medical condition of the patient 104 (e.g., hypothermia, trauma, etc.), or any combination thereof. In an embodiment, based on the input, the computing system may adjust the rotating valve to provide the volume of output air 110 to the patient 104.

In an embodiment, the computing system may be configured to control the rotating valve based on a breathing cycle, such as to provide output air 110 at a determined rate. In an embodiment, the breathing cycle may be pre-determined and stored in the memory of the STEAM device 100. Thus, the STEAM device 100 may be configured to prevent hyperventilation, such as by preventing the operator 108 from providing output air 110 to the patient 104 at a rate faster than the patient 104 can effectively process it. In an embodiment, the operator 108 may adjust the breathing cycle, such as via an input on the user interface. In at least one example, the breathing cycle may include an output of output air 110 (e.g., one breath) every five to six seconds.

In an embodiment, the computing system may control the rotating valve between an open position and closed position. In an embodiment, in the open position, at least a portion of the ambient air 102 may be mixed with pre-heated air from the furnace to generate the output air. In an embodiment, in the open position, at least a portion of the ambient air 102 may be shunted to the furnace section to generate the pre-heated air (e.g., furnace air), such as to refill the furnace after the pre-heated air has mixed with ambient air to generate the output air 110. As discussed above, the furnace may be heated by the wires in the furnace section. The computing system may rotate the valve toward a closed position to cease providing air to the patient, such as during an exhalation cycle. The computing system may determine the amount and/or rate at which to control the rotating valve based on the amount of furnace air needed to raise the temperature of ambient air 102 and generate the output air 110.

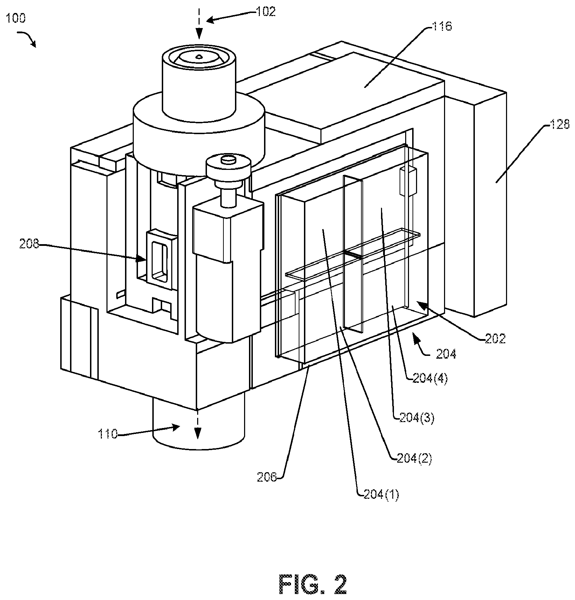

In an embodiment, the STEAM device 100 may include a first battery (FIG. 2) configured to provide power to one or more components (e.g., wires, humidifier, rotating valve, etc.). The first battery may include, but is not limited to, a voltage between 10-20V and a discharge rate of 50-200 milliamperes/hour. In an embodiment, the first battery may include a single cell battery. In an embodiment, the first battery may include two or more cells connected in series for discharge. In at least one example, the first battery may include four cells connected in series for discharge. In an embodiment, the first battery may be configured to power the component(s) based in part a discharge cycle. In such an embodiment, the discharge cycle may extend the service life of the STEAM device 100. In an embodiment, the discharge cycle may coincide with the breathing cycle. For example, the first battery may be configured to provide power to the component(s) for one second for every five to six seconds, similar to a breathing cycle.

In an embodiment, the STEAM device may include a second battery (FIG. 2). The second battery may be configured to charge one or more cells of the first battery, such as during times when the first battery is not discharging to power the component(s). In an embodiment, the second battery may include, but is not limited to, a voltage between 3-5V. In an embodiment, the second battery may be connected to the cell(s) of the first battery for charging. In at least one example, the second battery may be connected to two or more cells of the first battery in a parallel configuration for charging. In an embodiment, the computing system may determine the discharge cycle. In an embodiment, the discharge cycle may be based on the breathing cycle. In an embodiment, the discharge cycle may be determined based on a time needed to charge the first battery to a threshold charge (e.g., 80%, 85%, 90%, 95% charge or greater). In at least one example, the discharge cycle may include one second of discharge and five to six seconds of charge.

In an embodiment, the computing system may be configured to switch the first battery from a first circuit associated with discharging in series to a second circuit associated with charging in parallel. In an embodiment, the computing system may send a signal to a mechanical switch, causing the mechanical switch to switch the first battery from the first circuit to the second circuit. In an embodiment, the first circuit may include a circuit connecting the first battery to the component(s) of the STEAM device 100 and the second circuit may include a circuit connecting the first battery to the second battery for charging.

In an embodiment, the first battery and/or second battery may be a lithium-polymer or lithium-ion rechargeable battery. In order to make the device compact and lightweight, while maximizing operational time, the device may incorporate one or more first high-voltage, low-capacity batteries or cells arranged in series that are discharged during electrical heating and humidification during inhalation and are then shut off during a longer exhalation phase. The device may utilize one or more second low-voltage high-capacity batteries or cells to power other electrical components, such as the sensors and displays that require constant low current power consumption. During exhalation, when the one or more first high-voltage, low-capacity batteries or cells are not connected to the heating and humidification components, the one or more second low-voltage, high-capacity batteries or cells utilize a charging circuit, such as a boost converter, to recharge the one or more first batteries.

FIG. 2 illustrates a rear perspective view of the STEAM device 100. As described above, the STEAM device 100 may include a first battery 202 configured to provide power to one or more components of the STEAM device 100, such as the top display 116, the humidifier cartridge 128, the heating wires, to name just a few. In an embodiment, the first battery 202 may power the component(s).

In an embodiment, the first battery 202 may include a single cell 204 battery. In an embodiment, the first battery 202 may include two or more cells 204. As illustrated in FIG. 2, the first battery 202 includes four cells 204, a first cell 204(1), a second cell 204(2), a third cell 204(3), and a fourth cell 204(4). In an embodiment, the cells 204 may be connected in series for discharge to the one or more components of the STEAM device. In such an embodiment, the first battery 202 may provide a combined voltage of each of the cells 204.

In an embodiment, a computing system of the STEAM device 100 may be configured to switch the first battery 202 from a first circuit associated with discharging the first battery 202 to power the component(s) to a second circuit associated with charging the cells 204 of the first battery 202. In an embodiment, the second circuit may connect the first battery 202 to a second battery 206 for charging. In such an embodiment, the second circuit may connect the second battery 206 to the first cell 204(1), the second cell 204(2), the third cell 204(3), and the fourth cell 204(4) in parallel for charging.

In an embodiment, the first battery 202 and/or the second battery 206 may be detachably coupled to the STEAM device 100. In such an embodiment, the first battery 202 and/or the second battery 206 may be configured to be removed and/or replaced. In an embodiment, a user interface of the STEAM device 100 may provide an indication of battery charge associated with the first battery 202 and/or the second battery 206, such as via the top display 116. In an embodiment, the user interface may provide an indication of a time to replace the first battery 202 and/or the second battery 206. In an embodiment, the computing system may determine that the voltage associated with the first battery 202 and/or the second battery 206 has dropped below a threshold voltage. In such an embodiment, the computing system may cause the indication of the time to replace the first battery 202 and/or the second battery 206 based on the voltage associated therewith dropping below the threshold voltage.

In an embodiment, the computing system may cause first battery 202 to switch between active circuits (e.g., closed circuit for energy transfer) based on a discharge cycle, such as via a mechanical switch. In an embodiment, the discharge cycle may be pre-determined, such as that stored in a memory of the STEAM device 100. In an embodiment, the discharge cycle may be determined based on the breathing cycle. In such an embodiment, the discharge cycle may be the same or substantially the same as (e.g., within 0.1 seconds, 0.3 seconds of) the breathing cycle. In an embodiment, data corresponding to the breathing cycle and/or the discharge cycle may be input via the user interface, such as on the top display 116 or a front display, as described above. In an embodiment, data corresponding to the breathing cycle and/or discharge cycle may be input from an external computing device, such as during an initial set-up of the STEAM device 100, a software update, or the like.

In an embodiment, the STEAM device 100 may include an external computing device port 208 via which the external computing system may be connected. In an embodiment, the external computing device port 208 may include a universal serial bus (USB) port, a micro-USB port, a PS/2 port, a serial port, or the like. In an embodiment, a user (e.g. operator 108 or other user) may connect the external computing device to the STEAM device 100 via the port to transfer data between the devices, such as in an initial set-up or update of the STEAM device 100.

FIG. 3A illustrates an exploded view of example components of the STEAM device 100. In an embodiment, one or more components of the STEAM device 100 may be manufactured via traditional manufacturing techniques. In an embodiment, the component(s) may be manufactured by 3-D manufacturing techniques, casting, molding, forming, machining, composite manufacturing, and/or any other method of manufacturing. In an embodiment, the component(s) of the STEAM device 100 may include a metal material (e.g., aluminum, steel, stainless steel, titanium, iron, alloys thereof, etc.), a plastic material (e.g., high-density polyethylene, acrylic, melamine, polycarbonate, etc.), a composite material (e.g., fiberglass, carbon fiber, etc.), or combinations of the foregoing.

In an embodiment, the component(s) described herein may be couple to one another utilizing one or more coupling mechanisms, such as screws, bolts, rivets, snap-fit connectors, rotating connectors, and the like. In an embodiment, one or more of the component(s) may be detachably coupled to the STEAM device 100. Thus, the component(s) may be removed and replaced. For example, the humidifier cartridge 128 may be detachably coupled, enabling a user, such as an operator 108 to remove an empty humidifier cartridge 128 and replace it with another, full humidifier cartridge 128.

In an embodiment, the STEAM device 100 may include a user interface casing 302. In an embodiment, the user interface casing 302 may include couplings and/or mounts for one or more components associated with a user interface in which an operator may interact with the STEAM device. The user interface casing 302 may include coupling and/or mounts for one or more of the front display 114 and/or the top display 116. In an embodiment, the front display 114 and/or the top display 116 may include touchscreens via which the operator may input settings for the STEAM device 100, such as a temperature for output air 110. Additionally or alternatively, the user interface casing 302 may include a control panel 120 via which the operator may input the settings for the STEAM device 100. In an embodiment, the user interface casing 302 may include a power controller 112 via which the operator may turn the STEAM device 100 on and off and/or dim one or more of the front display 114 and the top display 116 and/or one or more indicator lights, such as indicator lights 118.

In an embodiment, the user interface casing 302 may have coupled thereto a front cover configured to protect one or more components of the user interface casing 302 (e.g. front display 114, control panel 120, etc.). In an embodiment, the front display cover may include indictor lights, such as indicator lights 118. In an embodiment, the front cover 304 may include covers for indicator lights located on the user interface casing 302. In an embodiment, the covers may include different colors, such as a red cover and a green cover. In such an embodiment, the bulbs associated with the indicator lights may be standard white bulbs, and the cover may modify a color that the operator views (e.g., green for ready to squeeze bag, red to indicate for the operator to not squeeze the bag).

As discussed above, the STEAM device 100 may be configured to modify one or more properties of ambient air to generate output air to provide to a patient, such as to prevent or treat hypothermia. In an embodiment, the STEAM device 100 may mix heated, humidified air with the ambient air to generate the output air. In an embodiment, the STEAM device 100 may include a furnace section 306 in which the heated, humidified air is generated. The furnace section 306 may include a furnace 308 and an insulator 310. In an embodiment, the insulator 310 may include a sleeve on insulation material in which the furnace 308 may be housed. In an embodiment, the insulator 310 may include a blanket of insulating material, such as a ceramic material, a fiberglass material, or other materials with insulating properties.

The furnace 308 may include a heating element (e.g., heating wires) configured to heat air located therein (ambient air shunted to the furnace section 306). In at least one example, the wires may include a Nichrome resistance wire mesh. In an embodiment, the computing system may determine an amount of energy to apply to the wires to heat air in the furnace based in part on the temperature of the ambient air. In an embodiment, the temperature of the ambient air may be determined based on input from a temperature sensor 312 located in the input section 126. In an embodiment, the computing system may determine an amount of energy to apply to the wires to heat air in the furnace based on sensor data received from one or more sensors 314 located in the furnace section 306. In an embodiment, the one or more sensors 314 may be configured to determine a temperature and/or humidity of the air in the furnace section 306. In an embodiment, the computing system may utilize the temperature in the furnace section 306 to determine an amount of heated furnace air to mix with the ambient air to generate the output air at the determined (e.g., input, pre-set, etc.) temperature.

In an embodiment, the computing system may utilize the sensor(s) 314 to determine an amount of water (e.g., mist) to input into the furnace 308 from the humidifier cartridge. In an embodiment, the computing system may utilize sensor data from a humidity sensor 316 located in the input section 126 to determine the amount of water to input into the furnace 308. In an embodiment, the computing system may determine an amount of energy to send to an ultrasonic transducer associated with the humidifier cartridge 128 to eject the determined amount of water into the furnace 308. In an embodiment, the computing system may utilize pulse width modulation to control the ultrasonic transducer.

In an embodiment, the computing system may determine the amount of energy to send to the wires in the furnace and/or to the ultrasonic transducer based in part on a discharge cycle of a battery, such as first battery 202. In an embodiment, the computing system may be configured to manage a discharge cycle of the battery, causing the battery to discharge during a first period of the cycle (e.g., 0.9 seconds, 1 second, 1.5 seconds, etc.) and charge during a second period of the cycle (e.g., 5 seconds, 6 seconds, 6.1 seconds, etc.). In an embodiment, during the first period, the battery may power one or more components of the STEAM device, such as the wires in the furnace 308, the humidifier cartridge 128, an electric motor 318 configured to rotate a rotating valve 320, an output valve 322, or any combination thereof.

Additionally, in an embodiment, the battery may power one or more sensors 314, the temperature sensor 312, the humidity sensor 316, a pitot system 324, and/or one or more sensors 326 configured to measure one or more properties of the output air, and/or the pitot system 324 (collectively "sensors"). Alternatively, one or more of the sensors 314, sensor 312, sensor 315, the sensor(s) 326, and/or the pitot system 324 may have associated therewith a separate power source (e.g., independent of the battery) configured to provide power to one or more of the sensors. In an embodiment, the battery may be configured to power one or more components of the user interface casing 302 (e.g., front display 114, top display 116, control panel 120, etc.) based on the discharge cycle. In an embodiment not shown, one or more of the components of the user interface casing 302 may include a separate power source configured to provide power to the component(s) of the user interface casing 302. In an embodiment, the sensors and the component(s) may be powered by the same or a separate power source. In such an embodiment, the sensors and the component(s) may consistently receive power (e.g., during the first cycle and the second cycle) while the STEAM device 100 is powered on, such as by turning the STEAM device 100 on at the power controller 112.

As discussed above, the air in the furnace 308 may include ambient air that is shunted to the furnace 308 for pre-heating. In an embodiment, the computing device may power the motor 318 to rotate the rotating valve 320 an amount and at a rate determined by an input from the user and/or one or more properties of the ambient air (e.g., temperature, pressure, humidity, etc.). In an embodiment, the ambient air may enter into the STEAM device via the input section 126, traveling through a filter 328 configured to filter particulate matter from the air. In an embodiment, the filter may be housed in a valve cap 330. The valve cap may cover one or more components of the input section 126. In an embodiment, the valve cap 330 may house the temperature sensor 312 and/or the humidity sensor 316.

In an embodiment, the rotating valve 320 may be substantially sealed by the rotating valve bearing 332, the stationary valve fans 334, and/or the rotating valve fans 336. In an embodiment, the rotating valve fans 336 may rotate with a valve sleeve 338 from a closed position to an open position. In an embodiment, an amount of rotation and/or a speed (rate) of rotation may be determined by the computing device utilizing the techniques described above, such as based on one or more properties of the ambient air. For example, when the valve is at least partially opened, the rotating valve 320 may permit a portion of the heated air in the furnace 308 (e.g., furnace air) to mix with the ambient air to generate the output air at the determined temperature and/or humidity.

In an embodiment, the rotating valve 320 may be configured to permit an amount of ambient air to travel directly to the patient prior to the rotating valve 320 opening. The amount of ambient air may act as a safety barrier to prevent hot air from the furnace reaching the patient without sufficiently mixing with the ambient air. The amount of ambient air may be, for example, 10-30 milliliters of air. In at least one example, the amount of ambient air may be approximately 20 milliliters. After the amount of ambient air is released to the patient, the ambient air may be mixed with furnace air heated during the first cycle of the discharge cycle (e.g., when energy is transferred from the battery (e.g., first battery) to at least the wires of the furnace 308). The computing system may cause the motor 318 to rotate the rotating valve 320 at a rate determined based on one or more temperatures, pressures, humidities, and/or other properties of air detected by the STEAM device (e.g., associated with ambient air, furnace air, output air, etc.). In an embodiment, one or more of the properties of the ambient air (e.g., pressure, velocity, etc.) may be determined utilizing the pitot system 324 based at least in part on static and dynamic pressures of the ambient air. In an embodiment, the pitot system 324 may include a pitot heater configured to provide heat to the pitot system to prevent the pitot tube and/or the static port from freezing. In an embodiment, the pitot heater may be activated at temperatures below a threshold temperature (e.g., 5°C, 30°F, etc.) and/or above a threshold humidity (e.g., 85%, 90%, etc.). The pitot system 324 may include a pitot tube and a pitot tube base 325.

In an embodiment, the furnace air heated and stored in the furnace may be mixed with ambient air to generate the output air at the determined temperature and humidity. In an embodiment, a portion of ambient air may be routed into the furnace to replace the pre-heated furnace air. In an embodiment, during the second cycle (e.g., when energy is not transferred from the battery to one or more components of the STEAM device 100), residual heat in the wires may pre-heat the air in the furnace 308. In such an embodiment, the STEAM device 100 may be configured to passively (e.g., during the second cycle based on residual heat) and actively (e.g., during the first cycle based on energy provided to the wires) pre-heat the furnace air for the STEAM device 100.

As discussed above, the computing system may send a signal to the motor 318 to cause the motor to rotate the rotating valve 320 a particular amount and/or at a particular rate based one or more of the properties of the air throughout the STEAM device 100 (e.g., ambient air, furnace air, output air, etc.). Additionally, the rotation of the rotating valve 320 may be controlled based on a volume of air to be provided to the patient. As discussed above, the volume of air may be pre-determined, such as a default amount based on an average tidal volume associated with breathing, or it may be determined based on input by the operator, such as patient data, for example, indicating an age and/or size of the patient. Additionally or alternatively, the computing system may close the rotating valve 320 based on a determination that a ventilation rate has exceeded a pre-determined ventilation rate. Thus, the computing system may prevent the operator from hyperventilating the patient. The safety features associated with the rotating valve 320 (e.g., preventing hyperventilation, preventing an excessive amount of output air to be provided to the patient, etc.) may prevent barotrauma and may ensure an adequate amount of ventilation is being provided to the patient. This will be especially useful for inexperienced personnel operating the device or with nonstandard patients, such as children or elderly individuals who have unique tidal volume demands and can be especially prone to barotrauma. The rotating valve 320 may additionally be utilized to provide output air at a determined temperature and/or humidity.

Rotating the rotating valve 320 may cause the ambient air to be mixed with heated, humidified furnace air, to generate the output air substantially at the determined temperature and humidity (e.g., within a threshold temperature and/or humidity). In an embodiment, the output air may be provided to the patient via a mask of a bag valve mask, such as mask 106(b) of BVM 106. In an embodiment, the output air may be provided to the patient via a ventilator mask. In an embodiment, the sensor(s) 326 located in the output section 130 may include a temperature sensor and/or a humidity sensor, configured to determine the temperature and humidity of the output air. In an embodiment, the computing device may utilize the sensor data from the sensor(s) 326 to determine whether to modify an amount of water ejected into the furnace, to modify an amount or rate of rotation of the rotating valve 320, or the like. In such an embodiment, the computing system may continually monitor the properties of the output air to determine modifications to the system to ensure the patient receives output air substantially at the determined temperature and/or humidity.

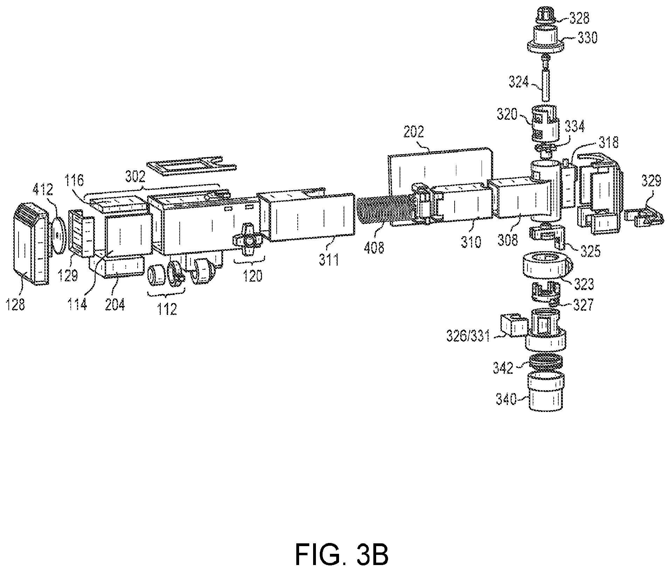

In an embodiment, the sensor(s) 326 may include a carbon dioxide sensor 331 (FIG. 3B). In an embodiment, the computing system may cause a carbon dioxide reading to be presented to the operator, such as via a front display 114 and/or a top display 116, based on sensor data. In such an embodiment, the sensor(s) 326 may enable the operator to monitor for capnography. In an embodiment, the carbon dioxide data presented on the display(s) may include end-tidal carbon dioxide waveform. Although illustrated as being housed in an output valve sleeve 340, this is merely for illustrative purposes, and the sensor(s) 326 may be located proximate other components of the output section. In at least one example, at least the carbon dioxide sensor of the STEAM device 100 may be located downstream of the output valve 322.

As discussed above, the STEAM device 100 may include an output valve 322. In an embodiment, the output valve 322 may facilitate output air going to the patient to be inhaled by the patient and/or exhaled air from the patient being expelled from the STEAM device (e.g., without entering the furnace 308). The output valve 322 may help prevent biomatter from entering the heating furnace. In an embodiment, an output valve spring 342 may enable the exhaled air to be expelled from the STEAM device 100. The output valve may also help prevent a patient from asphyxiating if the air input is closed or if the device loses power, as a "fail open" safety feature.

In at least one embodiment, the output valve 322 and/or the output valve spring 342 may be configured to maintain a determined positive end-expiratory pressure (PEEP) in the lungs of the patient. In such an embodiment, the output valve 322 and/or the output valve spring 342 may be adjustable. In an embodiment, the computing device may cause the output valve 322 and/or the output valve spring 342 to be modified based on a determined positive end-expiratory pressure. In an embodiment, the positive end-expiratory pressure may be predetermined, such as that stored on in the memory of the STEAM device 100. In an embodiment, the positive end-expiratory pressure may be fixed (e.g., not modified). In an embodiment, the positive end-expiratory pressure may be input via a user interface of the STEAM device 100 and/or by a manual selector 323 (FIG. 3B).

In an embodiment, the device may use at least one temperature sensor and a carbon dioxide sensor to determine if there is any smoke and/or combustion products that might be generated from combustion of biomatter in a high oxygen environment. There should be no carbon dioxide passing by the carbon dioxide sensor during inhalation, so the detection of carbon dioxide during inhalation may indicate a combustion event. In such an event, the device may display an alert to the user and turn off the heating element to prevent further combustion. Additionally, if the at least one temperature sensor and the carbon dioxide sensor are out of predetermined range, which may indicate combustion or a fire is occurring within the furnace, the device can turn on the ultrasonic transducer to spray the furnace and/or heating element with liquid.

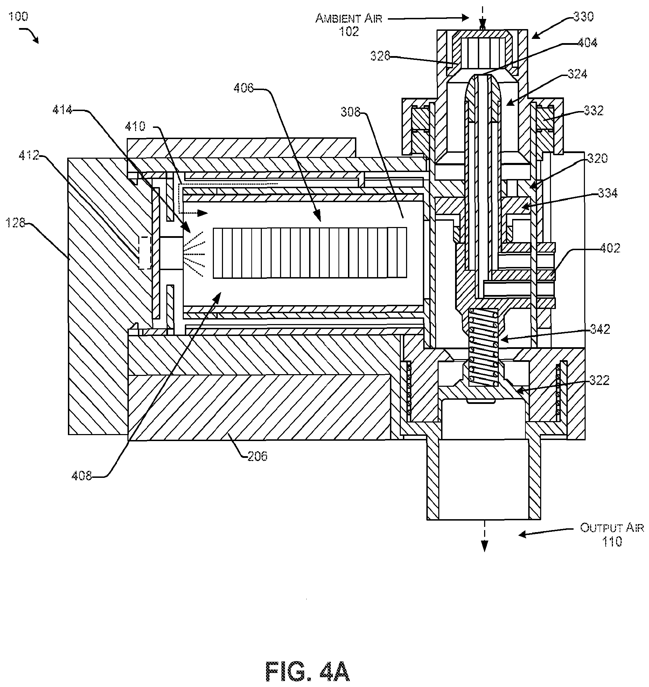

FIG. 3B illustrates an exploded view of example components of another embodiment of the STEAM device 100. Components and structure similar to that in FIG. 3A and FIGS. 4A-B are represented by the same reference numbers. The humidifier cartridge 128 may be removable attached to the housing via a slotted gate 129. The slotted gate 129 is openable and closeable. In a closed position, the slotted gate 129 creates a seal at one end of the device to allow air to travel through the furnace to the output section, even if no humidifier cartridge 128 is attached. The slotted gate 129 may fold inwardly to hold the humidifier cartridge in place when it is attached to the device.

The furnace section 306 includes furnace 308, heating element 408, and insulator 310, which acts as a heat shield for the heating element. The device may also include a heat shield 311. In an embodiment, the insulator 310 may be connected to any or all circuits of the device and therefore can transfer a set amount of heat to the device to keep it functioning in low temperature environments.

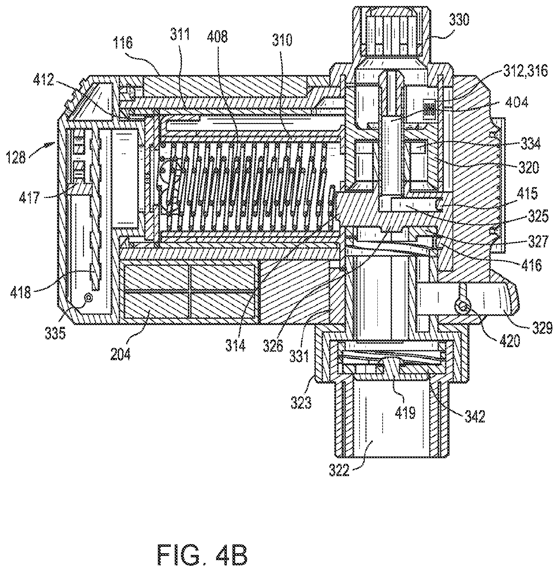

The device may include an air splitter element 327 (e.g., a valve or sliding plunger). The air splitter element 327 prevents CO₂ and/or biomatter from the patient from entering the device, thereby avoiding rebreathing and combustion hazards. Positive pressure during forced inhalation to the patient pushes air splitter element 327 downwards sealing an exhalation port 329. During exhalation, the air splitter element seals off the device and allows CO₂ to exit the exhalation port 329. In an embodiment, a dynamic pressure sensor (FIG. 4B) may be used to calculate the input air velocity during inhalation and can be used to sense if the patient is trying to breathe spontaneously, which can trigger the device to open rotating valve 320. FIG. 3B also shows PEEP mounting component.

FIG. 4A illustrates a cross-sectional view of a thermogenic airway emergency management device 100 (STEAM device 100). As discussed above, the STEAM device 100 may be configured to input ambient air with one or more properties (e.g., temperature, humidity, pressure, etc.) and modify one or more of the properties to generate output air 110. The output air 110 may be provided to a patient via a mask of a BVM 106, such as mask 106(b), or a ventilator mask.

In an embodiment, the ambient air may first pass through a filter 328 of the STEAM device 100. The filter may be configured to filter out particulate matter (e.g., particles of dirt, leaves, etc.) that may disrupt the flow of air through the STEAM device 100 and/or potentially choke the patient. In an embodiment, the filter 328 may include a filter cap configured to be housed within a valve cap 330. In an embodiment, the filter cap may be configured proximate one or more sensors, such as temperature sensor 312 and/or humidity sensor 316. In such an embodiment, a computing system of the STEAM device 100 may be configured to determine the temperature and pressure of the ambient air proximate an input.

In an embodiment, the ambient air 102 may pass through a pitot system 324. As described above, the pitot system 324 may be configured to measure a dynamic pressure and/or a static pressure associated with the ambient air 102. In an embodiment, the pitot system 324 may include a pressure channel 402 configured to measure the static pressure of the ambient air 102. In an embodiment, the pitot system 324 may include a pitot tube 404 configured to measure the dynamic pressure of the ambient air 102. In an embodiment, the pitot system 324 and/or the computing device may determine a velocity of the ambient air 102 based at least in part on the dynamic pressure and the static pressure of the ambient air 102. In an embodiment, the pitot system 324 may include a pitot heater configured to heat at least one of the pitot tube 404 and/or an opening of the pressure channel 402 to ensure accurate pressure measurements are captured therefrom. In an embodiment, the pitot heater may be activated below a threshold temperature and/or above a threshold humidity. In an embodiment, the pitot system may include a static pressure sensor 415 and a dynamic pressure sensor 416 (FIG. 4B).

In an embodiment, the computing system may utilize the velocity, pressure determined based on data from the pitot system 324, and the humidity, temperature of the ambient air 102 to determine an amount and/or a rate of rotation of the rotating valve 320 for mixing ambient air 102 with furnace air 406 stored in the furnace 308. As discussed above, the furnace air 406 may include ambient air 102 that is heated based on passive and/or active heat provided by the heating wires 408. Although illustrated as spanning a fraction of the furnace 308 surface area, this is for illustrative purposes only and is not intended to be limiting. In an embodiment, the heating wires 408 may cover a greater or lesser surface area of the furnace 308. In at least one example, the heating wires 408 may substantially surround the surface area of the furnace 308, thereby heating the furnace air 406 substantially the same throughout the furnace 308.

In an embodiment, the furnace air 406 may be actively heated when ambient air 102 is routed into the furnace 308 during a discharge cycle of a battery such as first battery 202 (e.g., when the first battery sends energy to the heating wires 408). A non-limiting example of the flow of ambient air 102 into the furnace 308 is illustrated by flow 410. In an embodiment, the furnace air 406 may be passively heated when the first battery is not sending energy to the heating wires 408 (e.g., when the air is trapped in the furnace 308 proximate the heating wires 408), such as when the second battery 206 is charging the first battery. Thus embodiment, the heating wires 408 may transfer residual heat to the furnace air 406 without requiring energy from a battery. In an embodiment, the passive heating of the furnace air 406 may enable the system to generate output air at a reduced power output.

In an embodiment, during the discharge cycle, the computing device may send a signal to an ultrasonic transducer 412 associated with the humidifier cartridge 128 to eject water 414 in the form of a mist into the furnace 308. In an embodiment, the computing device may determine an amount of water 414 to eject into the furnace 308 based on at least one of the temperature and/or humidity of the ambient air, the temperature of the furnace air, the output air humidity, the output air temperature, or any combination thereof. In an embodiment, the computing device may cause the ultrasonic transducer 412 to eject the amount of water 414. In an embodiment, the water 414 may encounter the heating wires in the furnace 308 and may vaporize. Thus, the water 414 may convert into steam and humidify the furnace air 406.

As discussed above, the heated, humidified furnace air 406 may be mixed with ambient air 102 to generate output air 110 at a determined temperature and/or humidity. In at least one example, the determined humidity may be about 50-100% humidity. In an embodiment, the determined temperature may be a temperature between about 75°F and 104°F (about 24°C and 40°C). In an embodiment, the determined temperature of the output air 110 may be input by an operator, such as based on a medical condition of the patient. For example, based on a determination that the patient is a suffering from severe hypothermia, the operator may determine to set a determined temperature to 104°F (40°C), to allow for rapid rewarming of the patient. For another example, based on a determination that the patient is a drowning victim and should be rewarmed slower than a patient with severe hypothermia, the operator may set the determined temperature to 100°F (38°C).

In an embodiment, the STEAM device 100 may store one or more preset temperatures and/or humidities based on patient medical conditions. In such an embodiment, the preset values may be stored in a memory of the STEAM device 100 in association with the computing device. In an embodiment, the preset temperatures and/or humidities may be based on clinical practice guidelines, such as those published by the American Association for Respiratory Care, or the like. In an embodiment, the operator may input patient data (e.g., at least one of height, weight, gender, age, body temperature, medical condition, or any combination thereof, etc.) and the computing device may be configured to determine a temperature and/or humidity of the output air 110 based on the patient data.

In an embodiment, the computing device may determine an amount and/or a rate at which to rotate the rotating valve 320 to mix the ambient air 102 and the furnace air 406 to generate the output air 110 at the desired temperature. The output air 110 may be output to the patient via the output valve 322. In an embodiment, the output valve 322 may include a one-way valve, allowing air to exit, but not enter, the STEAM device 100. In an embodiment, the output valve 322 may include one or more vents for exhaled air from the patient to be vented from the STEAM device 100.

In an embodiment, the output valve 322 may be an adjustable valve. In an embodiment, the output valve 322 may include an output valve spring 342 that is adjustable. In an embodiment the computing system may be configured to adjust a tension on the output valve spring 342, such as to set a positive end-expiratory pressure in the lungs of the patient. In an embodiment, the computing system may be configured to adjust the tension on the output valve spring 342 based in part on an input from the operator. Thus, the operator may input a positive end-expiratory pressure into a user interface. In an embodiment, the tension in the output valve spring 342 may be set based on a default positive end-expiratory pressure associated with the STEAM device 100.

FIG. 4B illustrates a cross-sectional view of a thermogenic airway emergency management device 100 according to another embodiment. Components and structure similar to that in FIG. 3A-3B and FIG. 4A are represented by the same reference numbers. The humidifier cartridge 128 may comprise at least one channel, for example two channels, and a spring-loaded plunger 417. Thus, a constant pressure may be applied to a liquid (e.g., water, saline, etc.) within the cartridge, thereby allowing a greater useable volume of liquid than a gravity activated plunger or mechanism. In an embodiment, the device may include a humidifier cartridge heating element or wire 418 to prevent liquid in the humidifier cartridge from freezing. In an embodiment, the humidifier cartridge heating element or wire 418 may be applied within or on a wall forming one or both channels. The humidifier cartridge may also have a refill port 335 that allows a user to inject liquid and/or medicine into the humidification cartridge, for example, via a syringe. In an embodiment, the ultrasonic transducer forms a mist and sprays medicine with moisture to the patient. This feature allows the device to act as an ultrasonic nebulizer and could be useful in patients that require inhaled medications (e.g., steroids) to help reduce airway swelling and irritation which makes breathing more difficult (e.g., asthma).

The output section 130 or output valve 322 may comprise a valve spring 342 to maintain a determined positive end-expiratory pressure (PEEP) in the lungs of the patient. In an embodiment, the valve spring may be connected to a flap 419. Flap 419 is attached to the valve spring 342 to allow the flap to freely flex downward when air is going to the patient during inhalation. The flap is not able to freely flex upward during exhalation. Because the flap cannot flex upward without compressing the valve spring, the force of the compressed valve spring maintains a positive end-expiratory pressure for the patient.

In an embodiment, the exhalation port 329 may have at least one openable and closeable exhalation switch or flap 420, for example, two switches or flaps. In an embodiment, the at least one switch 420 may be adjusted manually or may be adjusted automatically based on a reading of the dynamic pressure sensor 416. During exhalation, the air splitter element 327 seals off the device and allows CO₂ to exit the exhalation port 329 via an open at least one switch or flap 420. In an embodiment, if one switch or flap 420 is locked or gets stuck in a closed position and the air is not flowing to the patient through the device, a second switch or flap 420 in the exhalation port 329 may move freely with air flow, which allows a patient to inhale through the exhalation port 329 and thus serves as a safety feature.

Air flow through the device may not be constant over time. In an embodiment, air input may occur for every inhalation of the patient, which may be spontaneous or forced respiration, and which may approximately occur around one 1 breath every 5-6 seconds. In an embodiment, electrical power to the heating element may only be applied when air is flowing through the device to save battery charge. In an embodiment, the furnace heats the input air to a desired output temperature over 1 second, but not to exceed a predetermined maximum temperature to prevent thermal injury to the patient. The temperature of the heating element is proportional to the amount of electrical current passing through. The lower the input temperature and the faster the input air velocity are, the higher the electrical power applied to the heating element must be to achieve a constant temperature output. Pulse Width Modulation (PWM) signaling may be used to rapidly turn on and off the heating element over an inhalation period to allow for control of output temperature with dynamically changing input variables. In an embodiment, during the approximately 5 seconds before the next breath occurs while the patient is exhaling and no air is flowing into the device, the electrical power may be shut off from the heating element. Even though no electrical power is running through the heating element, the residual temperature of the heating element may exceed 500°F (260°C) which will raise the temperature of the static residual air volume in the furnace greater than the maximum safe temperature tolerated by the patient. In order to prevent thermal injury from this superheated air, the rotating valve may isolate the furnace from the patient's airway to prevent leakage of this hot air to the patient.

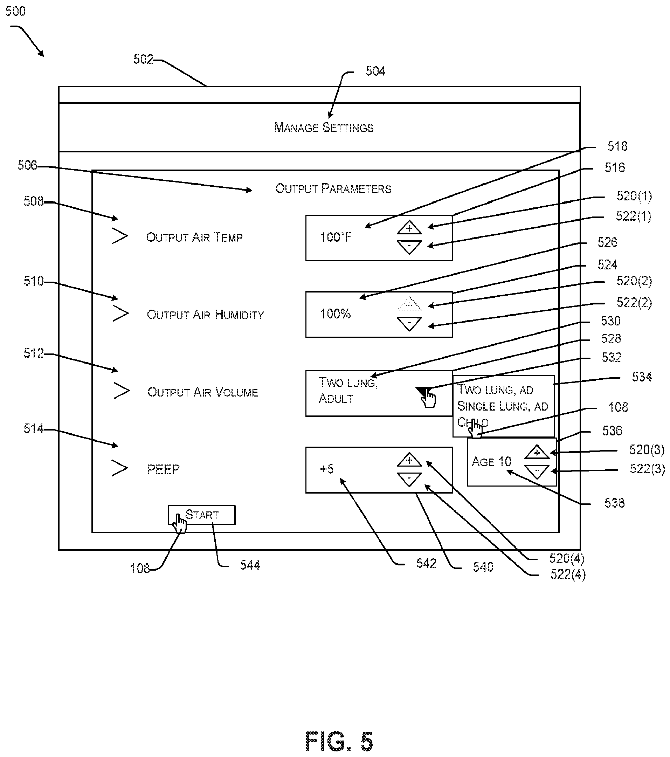

FIG. 5 illustrates an example user interface 500 associated with a thermogenic airway emergency management device, such as STEAM device 100, configured for receiving one or more inputs corresponding to one or more properties of air to be output via the STEAM device. In an embodiment, the user interface 500 may be presented via a display 502 of the STEAM device, such as front display 114 and/or top display 116.

In an embodiment, the user interface 500 may include a manage settings page 504. In an embodiment, the manage settings page 504 may provide a means by which an operator 108 (represented in FIG. 5 by a cursor on the user interface 500, such as that manipulated by a user) may input one or more output parameters 506 for the STEAM device. As illustrated in FIG. 5, the output parameter(s) 506 include, but are not limited to, at least one of an output air temperature 508, an output air humidity 510, an output air volume 512, or a positive end-expiratory pressure 514 (illustrated as PEEP). Though this is not intended to be so limiting, and the manage settings page 504 (or pages) may include a greater or lesser number of output parameters 506.

In an embodiment, one or more of the output parameters 506 may include default settings. In such an embodiment, a default setting may include a baseline of the respective parameter 506. For example, the default positive end-expiratory pressure 514 may include +5 (cm of water column). In an embodiment, one or more of the output parameters 506 may include a preset (e.g., fixed) value. Thus, a particular output parameter 506 may not be modified by the operator 108. For example, the STEAM device may be preset to generate output air at 100% humidity. In an embodiment, the default settings and/or the preset values may be stored in a memory associated with the STEAM device.

As illustrated in FIG. 5, the manage settings page 504 includes a temperature input window 516 in which the operator 108 may set and/or adjust a temperature 518 of the output air. In an embodiment, an initial value associated with the temperature 518 may include a preset and/or default setting. In an embodiment, the initial value may include a minimum value in a range of temperatures to which the STEAM device is limited. For example, the STEAM device may provide output air at a minimum of 100°F and a maximum of 103.5 °F (about 37°C to 40°C). In an embodiment, the temperature input window 516 may include a first increase control 520(1) enabling the operator 108 to increase the temperature 518 and a first decrease control 522(1) enabling the operator 108 to decrease the temperature 518.

In an embodiment, the manage settings page 504 may include a humidity input window 524 in which the operator 108 may set and/or adjust a humidity 526 of the output air. In an embodiment, an initial value associated with the humidity 526 may include a preset and/or default setting. In an embodiment, the humidity 526 may be fixed (e.g., not adjustable). In such an embodiment, a second increase control 520(2) and/or a second decrease control 522(2) associated therewith may not be selectable by the operator 108 (e.g., grayed out and/or may be withheld from presentation). In an embodiment, the second increase control 520(2) and/or a second decrease control 522(2) may be not selectable based on a determination that the humidity 526 is at a maximum or minimum value, respectively. For example, the maximum humidity 526 for the operator 108 to set may be 100% humidity. Based on a determination that the humidity 526 is set to the maximum value, the increase control 520(2) may not be selectable (e.g., grayed out).

In an embodiment, the manage settings page 504 may include an air volume input window 528 in which the operator 108 may set and/or adjust a volume of the output air. In an embodiment, the operator 108 may input a particular volume, such as 500 milliliters, 250 milliliters, or the like, in the volume input window 528. In such an embodiment, the computing system may receive the volume and may adjust a rotational valve of the STEAM device to permit the volume of output air to be output to the patient.

In an embodiment, the volume input window 528 may enable the operator 108 to input patient data 530 (e.g., adult, child, single lung, two lungs, etc.) corresponding to the patient. As illustrated in FIG. 5, the patient data 530 includes whether the patient is an adult or child and/or a number of lungs associated therewith. In an embodiment not shown, the patient data 530 may include any other data used to indicate a number of lungs (e.g., as an indication of tidal volume associated with breathing). In an embodiment, the computing system may determine a tidal volume associated with the patient and/or volume of output air to provide based on the patient data 530. In an embodiment, the tidal volume may include an average tidal volume for a patient corresponding to the patient data 530.

In an embodiment, the volume input window 528 may enable the operator 108 to input the patient data 530, such as via an associated keyboard, code, audio input via a speaker, or the like. In an embodiment, the volume input window 528 may include a selectable option 532 configured to facilitate the operator 108 modifying the patient data 530. As illustrated in FIG. 5, responsive to the operator 108 selecting the selectable option 532, a patient option window 534 may be presented via the manage settings page 504. The patient option window 534 may include options to select an adult with two lungs, an adult with a single lung, and a child, though this is merely for illustrative purposes and is not intended to be so limiting. In an embodiment, responsive to the operator 108 selecting the child option, an age input window 536 may be presented via the manage settings page 504. The age input window 536 may include an age 538 and a third increase control 520(3) and/or a third decrease control 522(3) to increase or decrease the age 538 of the patient. Based on the input provided via the patient option window 534 and/or the age input window 536, the computing system may determine an amount of output air to provide to the patient.

In an embodiment, the manage settings page 504 may include a positive end-expiratory pressure window 540 in which the operator 108 may set and/or adjust a positive end-expiratory pressure 542 associated with the output air. In an embodiment, an initial value associated with the positive end-expiratory pressure 542 may include a preset and/or default setting. In an embodiment, the positive end-expiratory pressure 542 may be fixed (e.g., not adjustable). In an embodiment, the positive end-expiratory pressure window 540 may include a fourth increase control 520(4) enabling the operator 108 to increase the positive end-expiratory pressure 542 and a fourth decrease control 522(4) enabling the operator 108 to decrease the positive end-expiratory pressure 542.

Additionally or alternatively, the user interface 500 may include an input for additional patient data (e.g., age, height, weight, condition, body temperature, etc.). In an embodiment, the computing system of the STEAM device may be configured to determine the one or more output parameters 506 based at least in part on the additional patient data. In such an embodiment, the computing system may store the operating parameters 506 in association with patient data in the memory associated with the STEAM device. For example, the operator 108 may input that an adult patient is suffering from severe hypothermia. The computing system may access the memory to determine an output temperature of 103°F (about 39°C), an output humidity of 100%, an output volume corresponding to two adult lungs, and a positive end-expiratory pressure of +5.

In an embodiment, the manage settings page 504 may include a start selectable option 544. The start selectable option 544 may indicate to the computing system that the settings input via at least one of the temperature input window 516, the humidity input window 524, the volume input window 528, or the positive end-expiratory pressure window 540 include determined properties for the output air. Additionally, the start selectable option 544 may provide an indication to the computing system to start generating the output air for the patient.

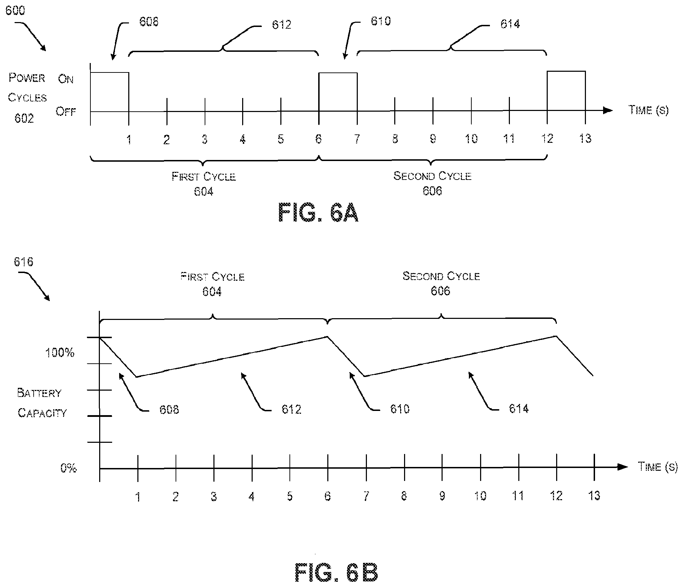

FIG. 6A illustrates an example chart 600 of power cycles 602 associated with a first battery, such as first battery 202 providing power to a device, such as STEAM device 100, based on the power cycles 602. In an embodiment, the chart 600 illustrates power cycles 602 over time. In an embodiment, the power cycles 602 may represent when the first battery is discharging power to one or more components of the device. In an embodiment, the power cycles 602 may be based on a breathing cycle. In an embodiment, the power cycles 602 may be determined based on a time to recharge the first battery.

As illustrated in FIG. 6A, the chart 600 includes two cycles, a first cycle 604 and a second cycle 606. During the first cycle 604, the first battery is configured to discharge power to the device at a first discharge 608 for, for example, one second and disconnect or be "off" for, for example, five seconds thereafter. During the second cycle 606, the first battery is configured to discharge power to the device at a second discharge 610 for one second and disconnect or be "off" for five seconds thereafter. Though illustrated as the same discharge pattern, the first cycle 604 and the second cycle 606 may have slightly different (e.g., within 0.3 seconds of one another) discharge patterns and are not limited to any particular time(s)).

In an embodiment, during the first discharge 608 and the second discharge 610, the first battery may be electrically coupled, via a first circuit, to the components of the device, such as the heating wires, the rotating valve, and the like. In an embodiment, two or more cells of the first battery may be connected in series and electrically coupled to the components of the device. In an embodiment, the computing system may determine the end of the first discharge 608 and may cause a mechanical switch to move from a first circuit to a second circuit associated with a second battery. In an embodiment, the second circuit may be associated with charging the first battery during an off period, such as first off period 612 and/or second off period 614. In an embodiment, the computing system may determine a start to the second discharge 610 and may cause the mechanical switch to move from the second circuit associated with the second battery to the first circuit for discharging power to the components of the device. After a period of time associated with the second discharge, the computing system may determine the end of the second discharge 610 and may cause the mechanical switch to move from the first circuit to the second circuit associated with the second battery.

FIG. 6B illustrates an example chart 616 of charging cycles associated with the second battery charging the first battery based on the power cycles. As discussed above, the first cycle 604 may include a first discharge 608 in which the first battery discharges power to the components of the device via the first circuit. In the chart 616, the first discharge is represented as a decrease in battery capacity. At the end of the first discharge 608, the computing system causes the first battery to switch from the first circuit associated with discharging power to the second circuit associated with the second battery. The second circuit may include a closed circuit between the second battery and the first battery. In at least one example, the second circuit may electrically couple the second battery to two or more cells of the first battery in parallel, providing a substantially equal charge to each of the cells of the first battery.

In an embodiment, during the first off period 612 the second battery may be configured to recharge the first battery to a battery capacity of substantially 100%. In an embodiment, the second battery may recharge the first battery to a battery capacity within a threshold battery capacity of the maximum (e.g., 80%, 85% 95%, 90%, etc.). In an embodiment, based on a determination that the second battery is unable to recharge the first battery to within the threshold battery capacity, the computing system may cause a notification to be presented to an operator of the device, such as operator 108, that battery power is low. In an embodiment, the notification may include an indication to replace the first battery with another battery.

Subsequent to a recharge of the battery during the first off period 612, the second cycle 606 may begin with a second discharge 610 in which the first battery discharges power to the components of the device via the first circuit. Similar to the first discharge 608, the second discharge 610 is represented on the chart 616 as a decrease in battery capacity. At the end of the second discharge 610, the computing system causes the first battery to switch from the first circuit associated with discharging power to the second circuit associated with the second battery. As illustrated, during the second off period 614, the second battery may be configured to recharge the first battery to a battery capacity of substantially 100%. In an embodiment, the second battery may recharge the first battery to a battery capacity within a threshold battery capacity of the maximum (e.g., 80%, 85% 95%, 90%, etc.).

In an embodiment, the computing system may be configured to cause the first battery to discharge power to the components of the device at a rate determined by the power cycles 602 (e.g., 1 second on, 5 seconds off, etc.) until the device is turned off, such as at a power controller. In an embodiment, during the off periods, such as first off period 612 and 614, the computing system may connect the first battery to the second circuit associated with the second battery, enabling the second battery to recharge the first battery. In such an embodiment, the recharging of the first battery by the second battery may extend the service life of the device, thereby increasing effectiveness thereof in remote, austere environments.

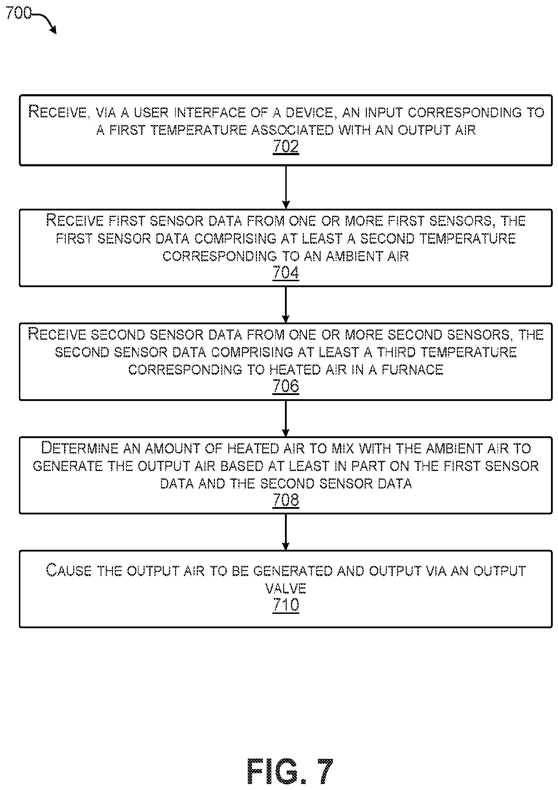

FIGS. 7-11 are flow diagrams illustrating example processes according to an embodiment. The processes of FIGS. 7-11 are illustrated as collections of blocks in logical flow diagrams, which represent a sequence of operations, some or all of which can be implemented in hardware, software or a combination thereof. In the context of software, the blocks may represent computer-executable instructions stored on one or more computer-readable media that, when executed by one or more processors, program the processors to perform the recited operations. Generally, computer-executable instructions include routines, programs, objects, components, data structures and the like that perform particular functions or implement particular data types. The order in which the blocks are described should not be construed as a limitation. Any number of the described blocks may be combined in any order and/or in parallel to implement the process, or alternative processes, and not all of the blocks need be executed. Further, in an embodiment, some or all of the operations illustrated in one or more of FIGS. 7-11 may be combined with some or all of the operations illustrated in others of FIGS. 7-11. For discussion purposes, the processes are described with reference to the environments, architectures and devices described in the examples herein, although the processes may be implemented in a wide variety of other environments, architectures and devices.

Various instructions, methods and techniques described herein may be considered in the general context of computer-executable instructions, such as program modules stored on computer-readable media and executed by the processor(s) herein. Generally, program modules include routines, programs, objects, components, data structures, etc., for performing particular tasks or implementing particular abstract data types. These program modules, and the like, may be executed as native code or may be downloaded and executed, such as in a virtual machine or other just-in-time compilation execution environment. Typically, the functionality of the program modules may be combined or distributed as desired in various implementations. An implementation of these modules and techniques may be stored on computer storage media or transmitted across some form of communication media.

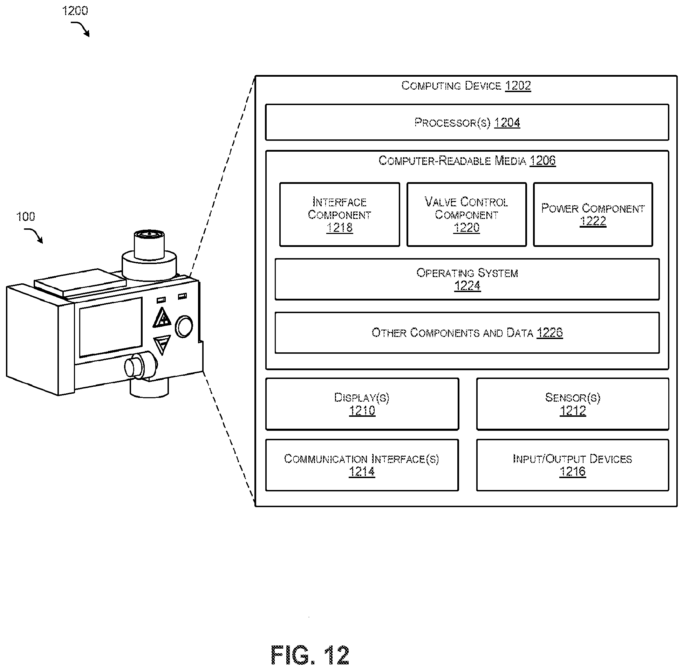

FIG. 7 is a flow diagram illustrating an example process 700 for modifying a temperature of ambient air to output air from a device at a particular temperature based on an input via a user interface. FIG. 7 is described in the context of the environments, device(s), and user interface(s) described above with reference to FIGS. 1-6B, but is not limited to such. The process 700 may be performed by a computing device, such as the computing system of the STEAM device 100, described with reference to FIGS. 1-6B. The process 700 may be performed by one or more components of the computing device, as described below with regard to FIG. 12.

At operation 702, the process 700 includes receiving, via a user interface of a device, an input corresponding to a first temperature associated with an output air. The output air may include air to be output by the device. The device may include a device associated with a system for thermogenic emergency airway management (STEAM), such as STEAM device 100. In an embodiment, the input may be received by a computing device of the device and stored in a memory thereof. In an embodiment, the input may be received via a touchscreen of the device and/or via a control panel, such as control panel 120.

At operation 704, the process 700 includes receiving first sensor data from one or more first sensors, the first sensor data comprising at least a second temperature associated with an ambient air. In an embodiment, the first sensor(s) may be located proximate an input section of the device, such as input section 126. In an embodiment, the first sensor(s) may include a temperature sensor, such as temperature sensor 312 configured to provide an indication of an outside air temperature in the ambient environment of the device. In an embodiment, the first sensor(s) may additionally include a humidity sensor configured to determine a humidity of the ambient air. In such an embodiment, the first sensor data may include the humidity of the ambient air.

In an embodiment, the first sensor(s) may include a pitot system, such as pitot system 324. In an embodiment, the pitot system may be configured to determine a static pressure and a dynamic pressure associated with ambient air, as it is input into the device. In an embodiment, a differential in pressure may result from ambient air being pushed into the device, such as via a bag of a BVM or a positive pressure air source.

In an embodiment, the computing system may receive data from the first sensor(s) and may be configured to determine the second temperature, a humidity, pressure, density, and/or a velocity of the ambient air.

At operation 706, the process 700 includes receiving second sensor data from one or more second sensors, the second sensor data comprising at least a third temperature corresponding to heated air in a furnace. The furnace, such as furnace 308, may be configured to heat ambient air during a discharge cycle of a battery and house heated air (e.g., furnace air 406) during a charge cycle when the STEAM device is not actively being powered. During the discharge cycle, heating wires, such as heating wires 408, may receive electrical energy from a battery and may convert the electrical energy into thermal energy to heat the air in the furnace to the third temperature.

At operation 708, the process includes determining an amount of heated air to mix with the ambient air to generate the output air based at least in part on the first sensor data and the second sensor data. In an embodiment, the computing system may utilize equations associated with the first law of thermodynamics to determine the amount of air to mix to generate the output air at the first temperatures. In an embodiment, the computing system may determine an amount and/or a rate to rotate a rotating valve to generate the output air. In such an embodiment, the amount and/or rate of rotation of the rotating valve may cause the amount of air to mix in the device.

At operation 710, the process includes causing the output air to be generated and output via an output valve. In an embodiment, the computing system may send a signal to a motor to cause the motor to rotate the rotating valve at the amount and/or rate determined based on the first sensor data, the second sensor data, and the first temperature. In an embodiment, prior to causing the rotating valve to rotate, the computing system may enable a small amount of ambient air (e.g., 20 milliliters, 25 milliliters, etc.) to bypass the heating system and be transferred directly to the patient. The small amount of ambient air may act as a cushion of cool air for the patient, to prevent the potential for burns caused by pre-heated air.

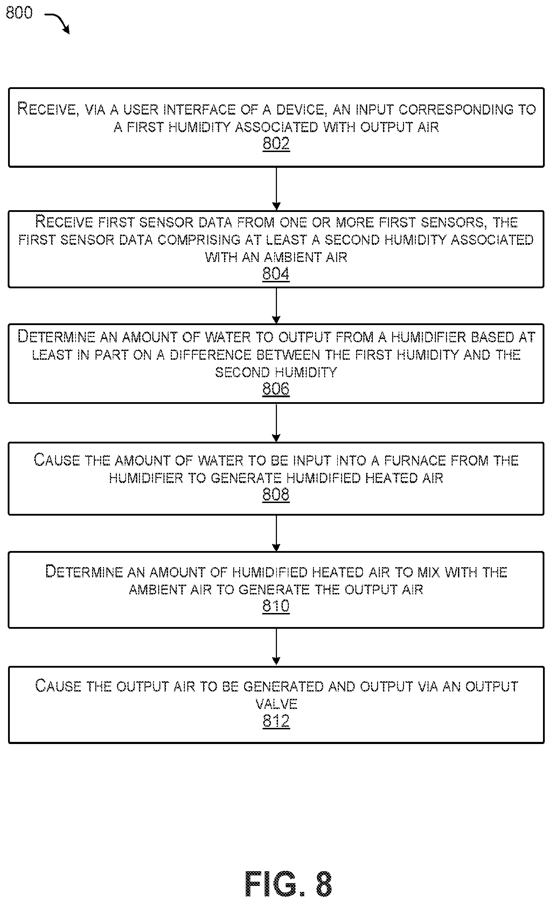

FIG. 8 is a flow diagram illustrating an example process 800 for modifying a humidity of ambient air to output air from a device at a particular humidity based on an input via a user interface. FIG. 8 is described in the context of the environments, device(s), and user interface(s) described above with reference to FIGS. 1-6B, but is not limited to such. The process 800 may be performed by a computing device, such as the computing system of the STEAM device 100, described with reference to FIGS. 1-6B. The process 800 may be performed by one or more components of the computing device, as described below with regard to FIG. 12.

At operation 802, the process 800 includes receiving, via a user interface of a device, an input corresponding to a first humidity associated with an output air. The output air may include air to be output by the device. The device may include a device associated with a system for thermogenic emergency airway management (STEAM), such as STEAM device 100. In an embodiment, the input may be received by a computing device of the device and stored in a memory thereof. In an embodiment, the input may be received via a touchscreen of the device and/or via a control panel, such as control panel 120.

At operation 804, the process 800 includes receiving first sensor data from one or more first sensors, the first sensor data comprising at least a second humidity associated with an ambient air. In an embodiment, the first sensor(s) may be located proximate an input section of the device, such as input section 126. In an embodiment, the first sensor(s) may include a humidity sensor configured to determine a humidity of the ambient air. In such an embodiment, the first sensor data may include the humidity of the ambient air. In an embodiment, the first sensor(s) may additionally include a temperature sensor, such as temperature sensor 312 configured to provide an indication of an outside air temperature in the ambient environment of the device.

At operation 806, the process 800 includes determining an amount of water (e.g., mist) to output from a humidifier based at least in part on a difference between the first humidity and the second humidity. In an embodiment, the humidifier may include a disposable humidifier cartridge, such as humidifier cartridge 128, comprising a volume of sterile water. In such an embodiment, the humidifier may be detachably coupled to the device. In an embodiment, the amount of water may be an amount of water per volume of air housed in a furnace, such as furnace 308 (e.g., a volume of the furnace). In at least one example, the amount of water may include a number of grams per liter of air in the furnace.

At operation 808, the process 800 includes causing the amount of water to be input into the furnace from the humidifier to generate humidified heated air. In an embodiment, the computing system may send a signal to an ultrasonic transducer of the humidifier to vibrate and cause water to spray into the furnace as a mist (e.g., fine mist). In at least one example, the transducer may be an ultrasonic piezoelectric transducer configured to vibrate at a resonance frequency between 110 kilohertz and 115 kilohertz. Pulse width modulation may be used to rapidly turn on and off the ultrasonic transducer to adapt to a dynamically changing input humidity.

In an embodiment, the computing system may send the signal to the ultrasonic transducer during a discharge cycle, such as when the components of the device are being powered. In an embodiment, the pre-heated air stored in the furnace may be output from the furnace and mixed with the ambient air during the discharge cycle (e.g., rotating valve rotates to generate the output air). In an embodiment, the water may be sprayed onto or proximate the heating wires of the furnace. In such an embodiment, the temperature of the heating wires may cause the water to vaporize in the furnace. In an embodiment, a cycle of ambient air may be routed into the furnace for pre-heating. The ambient air may mix with the water vapor to generate the humidified heated air.

At operation 810, the process 800 includes determining an amount of humidified heated air to mix with the ambient air to generate the output air. In an embodiment, the computing system may determine the amount of humidified heated air to mix with the ambient based on the first sensor data. In an embodiment, the computing system may determine the amount of humidified heated air to mix based on sensor data captured by one or more sensors in the furnace, such as a temperature sensor, a humidity sensor, or the like. In an embodiment, the computing system may determine the amount of humidified heated air based on the input received at operation 802.

In an embodiment, the computing system may utilize equations associated with the first law of thermodynamics to determine the amount of air to mix to generate the output air at the first humidity. In an embodiment, the computing system may determine an amount and/or a rate to rotate a rotating valve to generate the output air. In such an embodiment, the amount and/or rate of rotation of the rotating valve may cause the amount of air to mix in the device.

At operation 812, the process includes causing the output air to be generated and output via an output valve. In an embodiment, the computing system may send a signal to a motor to cause the motor to rotate the rotating valve at the amount and/or rate determined based on at least the first sensor data and the first humidity. In an embodiment, prior to causing the rotating valve to rotate, the computing system may enable a small amount of ambient air (e.g., 20 milliliters, 25 milliliters, etc.) to bypass the heating system and be transferred directly to the patient. The small amount of ambient air may act as a cushion of cool air for the patient, to prevent the potential for burns caused by humidified heated air in the furnace.

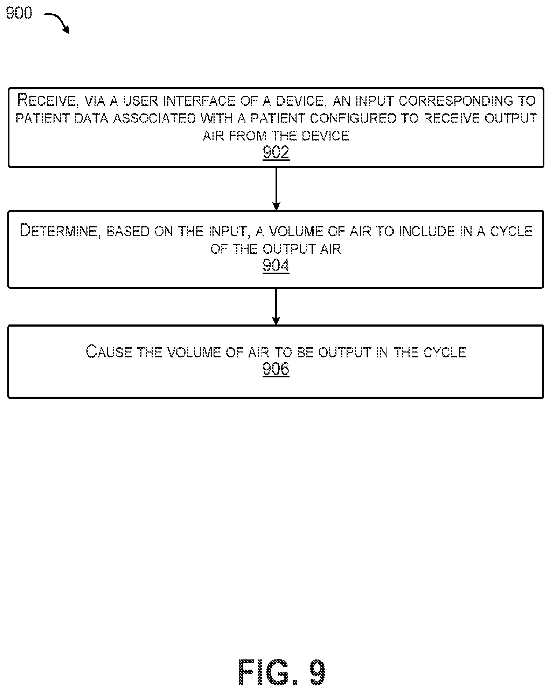

FIG. 9 is a flow diagram illustrating an example process 900 for outputting a particular amount of air from a device based on an input via a user interface. FIG. 9 is described in the context of the environments, device(s), and user interface(s) described above with reference to FIGS. 1-6B, but is not limited to such. The process 900 may be performed by a computing device, such as the computing system of the STEAM device 100, described with reference to FIGS. 1-6B. The process 900 may be performed by one or more components of the computing device, as described below with regard to FIG. 12.

At operation 902, the process 900 includes receiving, via a user interface of a device, an input corresponding to patient data associated with a patient configured to receive output air from the device. The device may include a device associated with a system for thermogenic emergency airway management (STEAM), such as STEAM device 100. In an embodiment, the input may be received by a computing system of the device and stored in a memory thereof. In an embodiment, the input may be received via a touchscreen of the device and/or via a control panel, such as control panel 120.

The patient data may include whether the patient has one or two lungs (e.g., as an indication of average tidal volume associated with breathing). In an embodiment, the patient data may include whether the patient is an adult or a child. In an embodiment, the patient data may include an age, height, weight, body temperature, and/or condition of the patient.

At operation 904, the process 900 includes determining, based on the input, a volume of air to include in a cycle of the output air. The cycle may correspond to a breathing cycle associated with the patient. The breathing cycle may include an inhalation of output air and an exhalation. In an embodiment, the inhalation and/or the exhalation may have times associated therewith. In such an embodiment, a combined time of the inhalation time and exhalation times may include a total time of a breathing cycle. In at least one example, the breathing cycle may be six seconds, with the inhalation time including one second and the exhalation 5 seconds. In an embodiment, the cycle time may be determined based on an average breathing rate for a patient. In an embodiment, the computing system may determine the cycle time based on the patient data. In an embodiment, the cycle time may include a predetermined (e.g., fixed, not adjustable) time, such as that stored in the memory associated with the computing system.

In an embodiment, the computing system may access a memory to determine the volume of air based on the input. In an embodiment, the volume may be based on an average tidal volume associated with a patient with the input patient data. For example, the patient data may include an indication that the patient is an adult with two lungs. Based on the patient data, the computing system may determine that the volume of air is about 300 to 600 milliliters, for example, 500 milliliters (e.g., +/- a threshold volume (e.g., 15 milliliters, 20 milliliters, etc.)). For another example, the patient data may include an indication that the patient is a six-year-old child. Based on the patient data may determine that the volume of air is about 100 milliliters.

Additionally, the computing system may determine at least one of a temperature or a humidity of the output air based at least in part on the patient data. In an embodiment, the temperatures and/or humidities may be stored in the memory, such as in a table or other data format, based on the patient data. For example, the patient data may include a condition of the patient (e.g., severe hypothermia, internal bleeding or other trauma, etc.). Based on the patient data, the computing system may determine the temperature and humidity of the output air to provide to the patient based on the patient data.

At operation 906, the process 900 includes causing the volume of air to be output in the cycle. In an embodiment, the computing system may send a signal to a motor to cause the motor to rotate a rotating valve at the amount and/or rate determined based on volume of air. The output air may then exit the device via an output valve and be inhaled by the patient via a mask, such as mask 106(b).

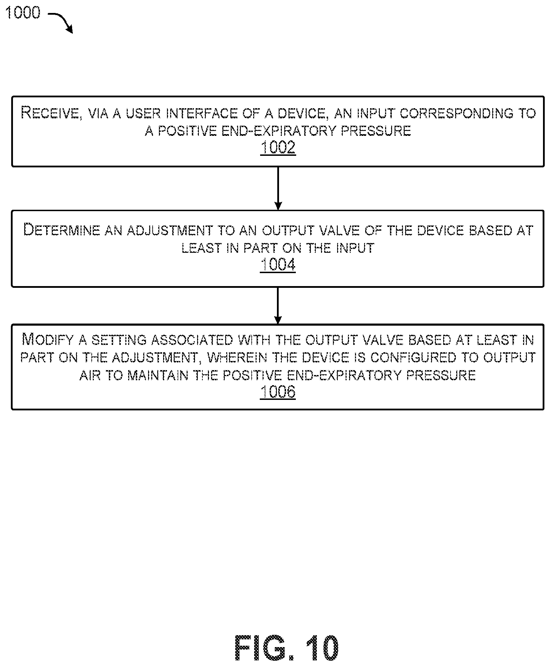

FIG. 10 is a flow diagram illustrating an example process 1000 for modifying a setting associated with an output valve of a device to maintain a positive end-expiratory pressure in a patient configured to receive air via the device. FIG. 10 is described in the context of the environments, device(s), and user interface(s) described above with reference to FIGS. 1-6B, but is not limited to such. The process 1000 may be performed by a computing device, such as the computing system of the STEAM device 100, described with reference to FIGS. 1-6B. The process 1000 may be performed by one or more components of the computing device, as described below with regard to FIG. 12.

At operation 1002, the process 1000 includes receiving, via a user interface of a device, an input corresponding to a positive end-expiratory pressure (PEEP). The PEEP may include a value representative of a pressure to be maintained in the lungs of a patient to which the device is attached, such as after exhalation. The patient may receive air from the device via a mask, such as mask 106(b).

In an embodiment, the input may be received by a computing system of the device and stored in a memory thereof. In an embodiment, the input may be received via a touchscreen of the device and/or via a control panel, such as control panel 120.

At operation 1004, the process 1000 includes determining an adjustment to an output valve of the device, such as output valve 322, based at least in part on the input. In an embodiment, the adjustment may include an adjustment to a spring of the output valve, such as output valve spring 342. In an embodiment, the adjustment may include an adjustment to the tension of the output valve spring.

At operation 1006, the process 1000 includes modifying a setting associated with the output valve based at least in part on the adjustment, wherein the device is configured to output air to maintain the positive end-expiratory pressure. In an embodiment, the computing system may cause the adjustment to be applied to the output valve and/or the output valve spring.

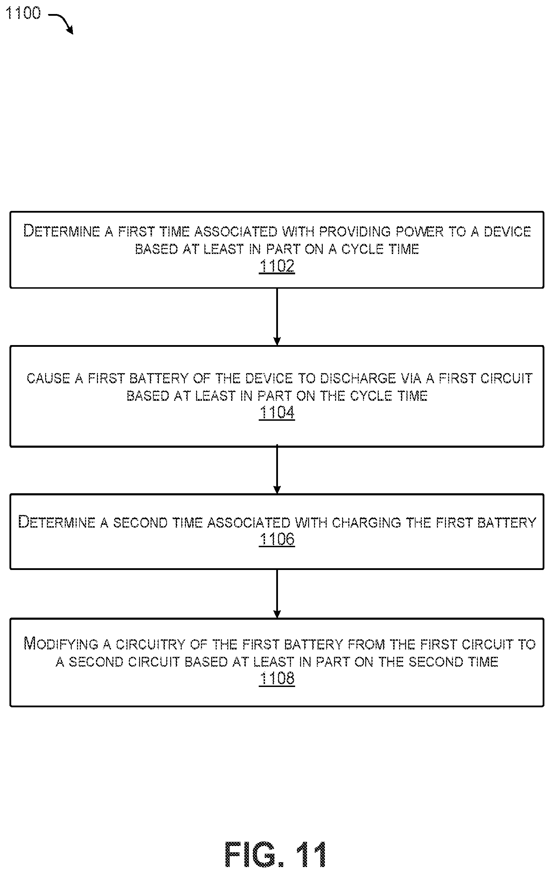

FIG. 11 is a flow diagram illustrating an example process 1100 for modifying a circuitry of a battery from a first circuit associated with discharging the battery to power a device to a second circuit associated with charging the battery based on a power cycle of a device. FIG. 11 is described in the context of the environments, device(s), and user interface(s) described above with reference to FIGS. 1-6B, but is not limited to such. The process 1100 may be performed by a computing device, such as the computing system of the STEAM device 100, described with reference to FIGS. 1-6B. The process 1100 may be performed by one or more components of the computing device, as described below with regard to FIG. 12.

At operation 1102, the process 1100 includes determining a first time associated with providing power to a device based at least in part on a cycle time. The cycle time may include a time associated with a breathing cycle. In an embodiment, the breathing cycle may include an inhalation and an exhalation, and times associated therewith. In an embodiment, the computing system may determine the cycle time based on an average breathing rate of a human. In an embodiment, the computing system may determine the cycle time based on patient data associated with a patient, such as an age, gender, condition, and the like. In such an embodiment, the cycle time may be specific to the patient.

In an embodiment, the cycle time may include a power cycle associated with a battery (e.g., first battery 202) providing power to one or more components of the device. In an embodiment, the power cycle may include a discharge time and a charge time. The discharge time may be associated with a time in which the battery is discharging power to the components of the device via a first circuit. The charge time may be associated with a time in which the battery is being charged by another battery (e.g., second battery 206). In an embodiment, the discharge cycle and the breathing cycle may substantially correspond. In such an embodiment, the discharge of power may correspond to an inhalation and the charging may correspond to an exhalation of a breathing cycle. In at least one example, the discharge time may be about one second and the charge time may be about five seconds.

At operation 1104, the process 1100 includes causing a first battery of the device to discharge via the first circuit based at least in part on the cycle time. In an embodiment, the computing system may determine the discharge time associated with the cycle. Based at least in part on the discharge time, the computing system may cause the first battery to be electrically connected to the components of the device (e.g., heating wires, rotating valve, etc.). In an embodiment, the computing system may determine that the first circuit is open. In such an embodiment, the computing system may cause the first circuit to be closed, in order to affect the discharge of power to the components of the device.

In an embodiment, the first circuit may include an electrical circuit electrically connecting the first battery to the components of the device, as described above. In an embodiment, the first circuit may electrically connect two or more cells of the first battery in series. In at least one example, the cell(s) of the first battery, when connected via the first circuit, may produce between 12 and 16 volts.

At operation 1106, the process 1100 includes determining a second time associated with charging the first battery. The second time may include a charge time associated with the cycle time. In an embodiment, the second time may be associated with the first battery being electrically connected to and receiving power from a second battery. In such an embodiment, the second battery may recharge the first battery.

At operation 1108, the process 1100 includes modifying a circuitry of the first battery from the first circuit to the second circuit based at last in part on the second time. In an embodiment, the computing system may determine that a current time is associated with the second time. Based at least in part on a determination that the current time is associated with the second time, the computing system may cause the second circuit to be closed, enabling the first battery to receive power from the second battery. In an embodiment, the computing system may cause a switch (e.g., a mechanical switch) to be adjusted, opening the first circuit and closing the second circuit. In such an embodiment, the computing system may cause the first battery to be disconnected from the components of the device and connected to the second battery.

In an embodiment, the second circuit may include an electrical circuit electrically connecting the first battery to the second battery, as described above. In an embodiment, the second circuit may electrically connect the two or more cells of the first battery to the second battery in parallel. In at least one example, the second battery may produce an equal or greater voltage than each of the cell(s) of the first battery, thereby enabling a charge thereof. In at least one example, the second battery may include a voltage in a range of about 3 and 5 volts.

FIG. 12 depicts an example block diagram illustrating a system 1200 for providing thermogenic emergency airway management, as discussed herein. The system may include an example computing device 1202 configured to cause at least one property associated with ambient air to be modified in order to prevent or treat hypothermia. As illustrated the computing device 1202 may be a component of a STEAM device 100. The computing device 1202 may include any type of computing device configured to perform the functions according to the techniques described herein. In at least one example, the computing device 1202 may include a microcontroller of the STEAM device 100.

In the illustrated example, the computing device 1202 includes at least one processor 1204, at least one memory 1206 (illustrated as computer-readable media), one or more displays 1210, such as front display 114 and/or top display 116, one or more sensors 1212, such as sensors 312, 314, 316, and/or 326, one or more communication interfaces 1214, and one or more input/output (I/O) devices 1216. In an embodiment not shown, the computing device 1202 may not include one or more of the display(s) 1210, the sensor(s) 1212, and/or the communication interface(s) 1214. Additionally, though described herein as including the displays 1210 and the sensors 1212, this is not intended to be so limiting, and the displays 1210 and/or the sensors 1212 may be coupled to the computing device 1202.

Each processor 1204 may itself comprise one or more processors or processing cores. For example, the processor 1204 may be implemented as one or more microprocessors, microcomputers, microcontrollers, digital signal processors, central processing units, state machines, logic circuitries, and/or any devices that manipulate signals based on operational instructions. In some cases, the processor 1204 may be one or more hardware processors and/or logic circuits of any suitable type specifically programmed or configured to execute the algorithms and processes described herein. The processor 1204 may be configured to fetch and execute computer-readable processor-executable instructions stored in the memory 1206.

Depending on the configuration of the computing device 1202, the memory 1206 may be an example of tangible non-transitory computer storage media and may include volatile and nonvolatile memory and/or removable and non-removable media implemented in any type of technology for storage of information such as computer-readable processor-executable instructions, data structures, program modules or other data. The memory 1206 may include, but is not limited to, RAM, ROM, EEPROM, flash memory, solid-state storage, magnetic disk storage, optical storage, and/or other computer-readable media technology. Further, in some cases, the computing device 1202 may access external storage, such as RAID storage systems, storage arrays, network attached storage, storage area networks, cloud storage, or any other medium that may be used to store information and that may be accessed by the processor 1204 directly or through another computing device or network. Accordingly, the memory 1206 may be computer storage media able to store instructions, modules or components that may be executed by the processor 1204. Further, when mentioned, non-transitory computer-readable media exclude media such as energy, carrier signals, electromagnetic waves, and signals per se.

The memory 1206 may be used to store and maintain any number of functional components that are executable by the processor 1204. In some implementations, these functional components comprise instructions or programs that are executable by the processor 1204 and that, when executed, implement operational logic for performing the actions and services attributed above to the computing device 1202. Functional components of the computing device 1202 stored in the memory 1206 may include an interface component 1218, a valve control component 1220, and a power component 1222. The interface component 1218 may present an interface on a display 1210 of computing device 1202 (e.g., such as the front display 114), to enable an operator, such as operator 108, to input one or more properties associated with output air and/or patient data associated with a patient configured to receive the output air, as described above. Further, the interface component 1218 may present an interface to inform the operator as to a status of the device (e.g., ready to output air to patient) and/or to instruct the operator when to provide the output air to the patient. Additional functional components stored in the memory 1206 may include an operating system 1224 for controlling and managing various functions of the computing device 1202 and for enabling basic user interactions with the computing device 1202.

The valve control component 1220 may be configured to determine an amount and/or a rate of rotation of a rotating valve, such as rotating valve 320, of the STEAM device 100. As discussed above, the amount and/or rate of rotation may be determined based on one or more properties of ambient air, furnace air, and determined properties associated with output air, such as those input by the operator or predetermined by the computing device 1202. In an embodiment, the valve control component 1220 may be configured to generate control signals to send to a motor of the STEAM device, such as motor 318. In an embodiment, the computing device 1202 may be configured to control the rotating valve based on the control signals. In an embodiment, the valve control component 1220 may cause the rotating valve to open based on the determined amount and/or rate of rotation. The valve control component 1220 may additionally cause the rotating valve to close, such as at an end of a breathing cycle.

In an embodiment, the valve control component 1220 may be configured to determine a volume of air to provide to the patient, such as in a breath of output air. As discussed above, the volume of air may be based on patient data, an average tidal volume of a human, or the like. In an embodiment, the valve control component 1220 may cause the rotating valve to be closed based on a determination that the volume of air has been provided to the patient and/or has passed through the rotating valve.

The power component 1222 may be configured to determine a power cycle associated with the STEAM device 100. The power cycle may include a discharge time and a charge time. In an embodiment, the power cycle may be associated with a breathing cycle. In such an embodiment, the discharge time may be associated with an inhalation and a charge time may be associated with an exhalation.

In an embodiment, the power cycle may include a fixed (e.g., not adjustable) cycle. In such an embodiment, the power component 1222 may access data associated with the power cycle, stored in the memory 1206. In an embodiment, the power cycle may be adjustable. In such an embodiment, the power component 1222 may receive the data associated with the power cycle from the interface component 1218, such as based on an input provided by the operator.

In an embodiment, the power component 1222 may be configured to switch a first battery of the STEAM device 100 between a first circuit associated with discharging power to components of the STEAM device 100 and a second circuit associated with charging the first battery. In at least one example, the power component 1222 may send a control signal to a mechanical switch, to switch from the first circuit to the second circuit, and vice versa. As discussed above, the first circuit may include a circuit in which two or more cells of the first battery discharge power to the components in series and the second circuit may include a circuit in which the two or more cells of the battery are charged in parallel by a second battery.

In addition, the memory 1206 may store data, data structures and the like, that are used by the functional components. For example, this data may include patient data, volume of air, predetermined device settings (e.g., output air properties, etc.), predetermined ranges of output air properties, and the like. Depending on the type of the computing device 1202, the memory 1206 may also optionally include other modules and data 1226, which may include programs, drivers, etc., and the data used or generated by the functional components. Further, the computing device 1202 may include many other logical, programmatic and physical components, of which those described are merely examples that are related to the discussion herein.

FIG. 12 further illustrates that the computing device 1202 may include (or be coupled to) the display(s) 1210 mentioned above. The display(s) 1210 may include a liquid crystal display, a plasma display, a light emitting diode display, an OLED (organic light-emitting diode) display, an electronic paper display, or any other suitable type of display able to present digital content thereon. In an embodiment, the display(s) 1210 may include a touch sensor associated with the display(s) 1210 to provide a touchscreen display configured to receive touch inputs for enabling interaction with a graphical user interface presented on the display 1210. Accordingly, implementations herein are not limited to any particular display technology.

The sensor(s) 1212 may include temperature sensors, humidity sensors, pressure sensors (e.g., pitot system 324), volume sensors, carbon dioxide sensors, rate sensors, timing devices, and/or any other sensors and/or sensing systems that can be utilized to perform the functions described herein. In an embodiment, one or more of the sensor(s) 1212 may be located in an input section, such as input section 126, a fumace section, such as fumace section 306, and/or an output section, such as output section 130, of the STEAM device 100. In such an embodiment, the computing device 1202 may be communicatively coupled to the sensor(s) 1212 and configured to receive sensor data therefrom. In an embodiment, the valve control component 1220 and/or the power component 1222 may process the sensor data from the sensor(s) 1212 and may perform one or more respective functions thereof based on the sensor data.

The one or more communication interface(s) 1214 may include one or more interfaces and hardware components for enabling communication with various other devices, such as over a network or directly. For example, communication interface(s) 1214 may enable communication through a wired connection, such as via a port, such as external computing device port 208. For another example, communication interface(s) 1214 may enable communication through one or more of the Internet, cable networks, cellular networks, wireless networks (*e.g*., Wi-Fi) and wired networks, as well as close-range communications such as Bluetooth^{®}, Bluetooth^{®} low energy, and the like, as additionally enumerated elsewhere herein.

The I/O devices 1216 may include indication lights, such as indicator lights 118, speakers, such as speaker 119, a microphone, and various user controls (*e.g*., buttons, a joystick, a keyboard, a keypad, etc.), a haptic output device, and so forth. In an embodiment, the I/O devices 1216 may provide an indication to the operator of a status of the STEAM device 100, such as when to provide air to the patient. For example, the I/O devices 1216 may include a speaker via which the interface component 1218 may cause a countdown timer to be emitted via the speaker indicating a countdown to providing output air to the patient. For another example, the I/O devices 1216 may include indicator lights indicating a status of the STEAM device 100, such as a red light to indicate that the STEAM device 100 is not ready to provide air and a green light to instruct the operator to provide the air.

While the aforementioned disclosure makes reference to user interactions via a user interface presented via the display 1210 of the computing device 1202, the user interface can be presented via any input/output device. As an example, the UI can be output via a speaker, and augmented reality projector, etc.

The foregoing is merely illustrative of the principles of this disclosure and various modifications can be made by those skilled in the art without departing from the scope of this disclosure. The above described examples are presented for purposes of illustration and not of limitation. The present disclosure also can take many forms other than those explicitly described herein. Accordingly, it is emphasized that this disclosure is not limited to the explicitly disclosed methods, systems, and apparatuses, but is intended to include variations to and modifications thereof, which are within the scope of the following claims.

As a further example, variations of apparatus or process limitations (*e.g*., dimensions, configurations, components, etc.) may be made to further optimize the provided structures, devices and methods, as shown and described herein. In any event, the structures and devices, as well as the associated methods, described herein have many applications. Therefore, the disclosed subject matter should not be limited to any single example described herein, but rather should be construed in breadth and scope in accordance with the appended claims.

### INDUSTRIAL APPLICABILITY

The present invention is directed to a device for preventing and/or treating hypothermia in a patient. The device described herein allows patients to receive advanced treatment for hypothermia in a pre-hospital setting. Accordingly, the device of the present invention may significantly decrease mortality rates in a pre-hospital setting, saving lives in a myriad of environments, such as austere environments, in low temperature climates or situations, on the battlefield, in the mountains, near water, and the like.

## Claims

1. A device (100) for treating and/or preventing hypothermia in a patient, comprising
an opening for ambient air;
a temperature sensor (312) configured to measure a temperature of the ambient air;
at least one battery (202);
a furnace section (306) comprising a furnace (308), a heating element (408) configured to heat air in the furnace section; a temperature sensor (314) to determine a temperature of air in the furnace section, and an insulator (310) in which the furnace (308) is housed;
a valve (320) configured to mix ambient air with pre-heated furnace air to form output air at an output air temperature based at least in part on data from the temperature sensor (312) configured to measure the temperature of the ambient air; and
an output for providing the output air to a patient.

2. The device according to claim 1, further **characterized by**:
a humidity sensor (316) configured to measure a humidity of the ambient air;
one or more output air sensors (326) configured to measure a temperature and a humidity of the output air; and/or
a carbon dioxide sensor (331) in an output section of the device.

3. The device according to claim 1, further **characterized by** a pitot system (324) comprising:
a pressure channel (402) to measure a static pressure of the ambient air ;
a pitot tube (404) to measure a dynamic pressure of the ambient air and a pitot heater configured to heat at least one of the pitot tube or an opening of the pressure channel.

4. The device according to claim 1, further **characterized by** a valve or sliding plunger (327) configured to:
seal an exhalation port (329) during inhalation of the patient; and
seal the device during exhalation of the patient and open the exhalation port (329).

5. The device according to claim 1, further **characterized by** an exhalation port (329) having at least one switch or flap (420) that is openable and closeable based at least in part on data from a dynamic pressure sensor (416).

6. The device according to claim 1, further **characterized by** a removably attachable humidifier cartridge (128) comprising ultrasonic transducer (412) configured to spray mist into the furnace section.

7. The device according to claim 6, **characterized in that** the humidifier cartridge (128) comprises at least one channel for the liquid and a spring-loaded plunger (417) configured to provide a constant pressure to the liquid and a heating element or wire (418) configured to prevent the liquid from freezing.

8. The device according to any one of claims 1-7, further **characterized by** an output section (130) having an output valve (322) and/or an output spring configured to maintain a positive end-expiatory pressure in lungs of the patient.

9. The device according to any one of claims 1-7, wherein the at least one battery is **characterized by** a first battery (202) configured to provide power to one or more components of the device during inhalation and a second battery (206) configured to charge the first battery during exhalation, wherein the first battery comprise two or more cells connected in series.

10. The device according to claim 1, **characterized in that** the opening is connectable to a bag (106a) or a positive pressure air source (132).

11. The device (100) according to any one of claims 1-10, further **characterized by**:
one or more processors (1204) ; and
one or more computer-readable media (1206) storing instructions executable by the one or more processors (1204) , wherein the instructions program the one or more processors to:
receive sensor data associated with one or more sensors;
determine an amount to open the valve to mix furnace air with ambient air based at least in part on sensor data, wherein the furnace air heats the ambient air to an output air temperature associated with the output air.

12. The device (100) according to claim 11, further **characterized by** a display (1210),
wherein the instructions further program the one or more processors (1204) to:
cause a user interface to be presented via the display (1210) of the device, the user interface enabling an operator of the device to input a property of the air; and
receive, via the user interface, the output air temperature associated with the output air.

13. The device (100) according to claim 11 or 12, **characterized in that** the instructions further program the one or more processors (1204) to:
determine a cycle associated with providing the output air to the patient, wherein the cycle comprises an inhalation of the air and an exhalation of at least a portion of the air from the patient;
determine a first period of time associated with the inhalation; and
cause an indication of the first period of time to be presented to an operator via a user interface of the device.

## Patentansprüche

1. Vorrichtung (100) zur Behandlung und/oder Vorbeugung von Unterkühlung bei einem Patienten, umfassend
eine Öffnung für Umgebungsluft;
einen Temperatursensor (312), der so konfiguriert ist, dass er die Temperatur der Umgebungsluft misst;
mindestens eine Batterie (202);
einen Ofenabschnitt (306), der einen Ofen (308), ein Heizelement (408), das zum Erwärmen von Luft im Ofenabschnitt ausgelegt ist, einen Temperatursensor (314) zur Bestimmung einer Temperatur der Luft im Ofenabschnitt und einen Isolator (310), in dem der Ofen (308) untergebracht ist; umfasst,
ein Ventil (320), das dazu ausgelegt ist, Umgebungsluft mit vorgewärmter Ofenluft zu mischen, um Ausgangsluft mit einer Ausgangslufttemperatur, die zumindest teilweise auf Daten des Temperatursensors (312), der dazu ausgelegt ist, die Temperatur der Umgebungsluft zu messen, basiert, zu erzeugen; und
einen Auslass zum Zuführen der Ausgangsluft zu einem Patienten.

2. Vorrichtung nach Anspruch 1, ferner **gekennzeichnet durch**:
einen Feuchtigkeitssensor (316), der so konfiguriert ist, dass er die Feuchtigkeit der Umgebungsluft misst;
einen oder mehrere Auslassluftsensoren (326), die so konfiguriert sind, dass sie eine Temperatur und eine Feuchtigkeit der Ausgangsluft zu messen; und/oder einen Kohlendioxidsensor (331) in einem Auslassabschnitt der Vorrichtung.

3. Vorrichtung nach Anspruch 1, ferner **gekennzeichnet durch** ein Pitot-System (324), das umfasst:
einen Druckkanal (402) zur Messung eines statischen Drucks der Umgebungsluft;
ein Pitotrohr (404) zur Messung eines dynamischen Drucks der Umgebungsluft, und eine Pitot-Heizung, die so konfiguriert ist, dass sie zumindest eines von Pitotrohr oder einer Öffnung des Druckkanals erwärmt.

4. Vorrichtung nach Anspruch 1, ferner **gekennzeichnet durch** ein Ventil oder einen Schiebekolben (327) so ausgebildet,
eine Ausatemöffnung (329) während der Einatmung des Patienten abzudichten; und die Vorrichtung während der Ausatmung des Patienten abzudichten und die Ausatemöffnung (329) zu öffnen.

5. Vorrichtung nach Anspruch 1, ferner **gekennzeichnet durch** eine Ausatemöffnung (329) mit mindestens einem Schalter oder einer Klappe (420), die zumindest teilweise auf der Grundlage von Daten eines dynamischen Drucksensors (416) geöffnet und geschlossen werden kann.

6. Vorrichtung nach Anspruch 1, ferner **gekennzeichnet durch** eine abnehmbar anbringbare Befeuchterkartusche (128), die einen Ultraschallwandler (412) umfasst, der so ausgelegt ist, dass er Nebel in den Ofenbereich versprüht.

7. Vorrichtung nach Anspruch 6, **dadurch gekennzeichnet, dass** die Befeuchterkartusche (128) mindestens einen Kanal für die Flüssigkeit und einen federbelasteten Kolben (417) umfasst, der so ausgelegt ist, dass er einen konstanten Druck auf die Flüssigkeit ausübt, sowie ein Heizelement oder einen Heizdraht (418), der so ausgelegt ist, dass er ein Gefrieren der Flüssigkeit verhindert.

8. Vorrichtung nach einem der Ansprüche 1 bis 7, ferner **gekennzeichnet durch** einen Auslassabschnitt (130) mit einem Auslassventil (322) und/oder einer Auslassfeder, die so ausgelegt ist, dass sie einen positiven endexspiratorischen Druck in den Lungen des Patienten aufrechterhält.

9. Vorrichtung nach einem der Ansprüche 1 bis 7, wobei die mindestens eine Batterie **gekennzeichnet ist durch** eine erste Batterie (202), die so ausgelegt ist, dass sie während der Einatmung eine oder mehrere Komponenten der Vorrichtung mit Strom versorgt, und eine zweite Batterie (206), die so ausgelegt ist, dass sie die erste Batterie während der Ausatmung auflädt, wobei die erste Batterie zwei oder mehr in Reihe geschaltete Zellen umfasst.

10. Vorrichtung nach Anspruch 1, **dadurch gekennzeichnet, dass** die Öffnung mit einem Beutel (106a) oder einer Überdruckluftquelle (132) verbindbar ist.

11. Vorrichtung (100) nach einem der Ansprüche 1 bis 10, ferner **gekennzeichnet durch**:
einen oder mehrere Prozessoren (1204); und
ein oder mehrere computerlesbare Medien (1206), die von dem einen oder den mehreren Prozessoren (1204) ausführbare Befehle speichern, wobei die Befehle den einen oder die mehreren Prozessoren so programmieren, um:
Sensordaten, die einem oder mehreren Sensoren zugeordnet sind, zu empfangen;
ein Öffnungsmaß des Ventils zum Mischen von Ofenluft mit Umgebungsluft zumindest teilweise auf den Sensordaten basierend zu bestimmen, wobei die Ofenluft die Umgebungsluft auf eine der Ausgangsluft zugeordnete Ausgangslufttemperatur erwärmt.

12. Vorrichtung (100) nach Anspruch 11, ferner **gekennzeichnet durch** eine Anzeige (1210), wobei die Befehle den einen oder die mehreren Prozessoren (1204) ferner so programmieren, dass sie: bewirken, dass eine Benutzeroberfläche über die Anzeige (1210) der Vorrichtung dargestellt wird, wobei die Benutzeroberfläche es einem Bediener der Vorrichtung ermöglicht, eine Eigenschaft der Luft einzugeben und über die Benutzerschnittstelle die der Abluft zugeordnete Ablufttemperatur zu empfangen.

13. Vorrichtung (100) nach Anspruch 11 oder 12, **dadurch gekennzeichnet, dass** die Anweisungen den einen oder die mehreren Prozessoren (1204) ferner so programmieren, um:
einen Zyklus zu bestimmen, der der Bereitstellung der Ausgangsluft für den Patienten zugeordnet ist, wobei der Zyklus ein Einatmen der Luft und ein Ausatmen zumindest eines Teils der Luft durch den Patienten umfasst;
eine erste Zeitspanne zu bestimmen, die der Einatmung zugeordnet ist; und
zu bewirken, dass eine Anzeige der ersten Zeitspanne einem Bediener über eine Benutzerschnittstelle der Vorrichtung präsentiert wird.

## Revendications

1. Dispositif (100) de traitement et / ou de prévention d'une hyperthermie chez un patient, comportant une ouverture pour de l'air ambiant ;
un capteur de température (312) conçu pour mesurer une température de l'air ambiant ;
au moins une batterie (202) ;
une section de combustion (306) comportant un four (308), un élément chauffant (408) conçu pour chauffer l'air dans la section four; un capteur de température (314) destiné à déterminer une température d'air dans la section de combustion, ainsi qu'un isolateur (310) dans lequel est installé le four (308) ;
une vanne (320) conçue pour mélanger l'air ambiant avec l'air préchauffé provenant du four pour constituer de l'air sortant à une température d'air sortant sur la base au moins en partie de données récupérées du capteur de température (312) conçu pour mesurer la température de l'air ambiant ; et
une sortie pour approvisionner un patient en air sortant.

2. Dispositif selon la revendication 1, **caractérisé par** ailleurs par :
un capteur d'humidité (316) conçu pour mesurer une humidité de l'air ambiant ;
un ou plusieurs capteur/s d'air sortant (326) conçu/s pour mesurer une température et une humidité de l'air sortant ; et / ou
un capteur de dioxyde de carbone (331) dans une zone de sortie du dispositif.

3. Dispositif selon la revendication 1, **caractérisé par** ailleurs par un système de Pitot (324) comportant :
un canal de pression (402) pour mesurer une pression statique de l'air ambiant ;
un tube de Pitot (404) pour mesurer une pression dynamique de l'air ambiant et un chauffeur de Pitot apte à chauffer au moins un des tubes de Pitot ou une ouverture du canal de pression.

4. Dispositif selon la revendication 1, **caractérisé par** ailleurs par une vanne ou un plongeur glissant (327) apte à :
obturer un orifice d'expiration (329) pendant l'inhalation du patient ; obturer le dispositif pendant l'expiration du patient et ouvrir l'orifice d'expiration (329).

5. Dispositif selon la revendication 1, **caractérisé par** ailleurs par un orifice d'expiration (329) comportant au moins un interrupteur ou volet (420) susceptible d'être ouvert ou fermé sur la base, au moins en partie, de données fournies par un capteur de pression dynamique.

6. Dispositif selon la revendication 1, **caractérisé par** ailleurs par une cartouche d'humidificateur amovible (128) comportant un transducteur ultrasonore (412) conçu pour pulvériser du brouillard dans la section de combustion.

7. Dispositif selon la revendication 6, **caractérisé en ce que** la cartouche d'humidificateur (128) comporte au moins un canal pour le liquide ainsi qu'un piston à ressort (417) apte à assurer une pression constante du liquide et un élément ou fil de chauffage (418) apte à éviter le gel du liquide.

8. Dispositif selon l'une quelconque des revendications 1 à 7, **caractérisé par** ailleurs par un tronçon de sortie (130) comportant une vanne de sortie (322) et / ou un ressort de sortie apte à maintenir une pression positive de fin d'exhalation dans les poumons du patient.

9. Dispositif selon l'une quelconque des revendications 1 à 7, dans lequel l'au moins une batterie est **caractérisée par** une première batterie (202) conçu pour alimenter en courant l'un ou les composants du dispositif pendant l'inhalation ainsi qu'une seconde batterie (206) apte à charger la première batterie pendant l'exhalation, dans lequel la première batterie comporte une ou plusieurs cellules connectées en série.

10. Dispositif selon la revendication 1, **caractérisé en ce que** l'ouverture est susceptible d'être reliée à un sac (106a) ou à une source d'air à pression positive (132).

11. Dispositif (100) selon l'une quelconque des revendications 1 à 10, **caractérisé par** ailleurs par :
un ou plusieurs processeurs (1204) ; et un ou plusieurs supports (1206) lisibles par ordinateur stockant les commandes exécutables par l'un ou les processeur/s (1204), dans lequel les commandes programment l'un ou les processeur/s de manière à :
recueillir des données de capteur associées à un ou plusieurs capteur/s ;
déterminer un montant menant à l'ouverture de la valve pour mélanger de l'air provenant du four avec l'air ambiant sur la base, au moins en partie, de données des capteurs, dans lequel l'air provenant du four chauffe l'air ambiant jusqu'à atteindre une température de sortie associée à l'air sortant.

12. Dispositif (100) selon la revendication 11, **caractérisé par** ailleurs par un écran (1210), dans lequel les commandes programment par ailleurs l'un ou les processeur/s (1204) de manière à :
ce qu'une interface utilisateur soit affichée à l'aide de l'écran (1210) du dispositif, l'interface utilisateur permettant à un opérateur du dispositif d'entrer une caractéristique de l'air ; et à obtenir, moyennant l'interface utilisateur, la température d'air sortant associée à l'air sortant.

13. Dispositif (100) selon la revendication 11 ou 12, **caractérisé en ce que** les commandes programment par ailleurs l'un ou les processeur/s (1204) de manière à :
déterminer un cycle associé au processus d'approvisionnement du patient en air sortant, dans lequel le cycle comporte une inhalation de l'air et une expiration d'au moins une partie de l'air par le patient ;
déterminer une première durée associée à l'inhalation ; et
à ce que la première durée soit affichée à un opérateur à l'aide d'une interface utilisateur du dispositif.
